# EUROPÄISCHE PATENTANMELDUNG

(11) **EP 4 545 067 A1**
(43) Veröffentlichungstag der Anmeldung: **30.04.2025**
(21) Anmeldenummer: 23205816.4
(22) Anmeldetag: 25.10.2023
(51) Int. Cl.: A61K 9/06, A01N 25/00, A01N 43/00, A01N 53/00, A61K 9/107, A61K 47/10, A61K 47/14, A61K 47/44, A61P 33/14

(54) **HOCHDOSIERTE DERMAL APPLIZIERBARE PERMETHRIN-ZUBEREITUNG MIT ERHÖHTEM DRUGLOAD IM ZIELKOMPARTIMENT ZUR VERBESSERTEN BEHANDLUNG VON EKTOPARASITEN BEFALL**

(71) Anmelder: Infectopharm Arzneimittel und Consilium GmbH, 64646 Heppenheim (DE)
(72) Erfinder: Wachall, Bertil, 69502 Hemsbach (DE); Vogt, Markus, 60438 Frankfurt (DE); ZOELLER, Phillipp, 64287 Darmstadt (DE)
(74) Vertreter: Fuchs Patentanwälte Partnerschaft mbB

(57) **Zusammenfassung**

Die vorliegende Erfindung betrifft eine halbfeste Creme-Zubereitung zur dermal-topischen Anwendung von / Behandlung bei Ektoparasiten, wobei die Creme-Zubereitung Wasser, ein oder mehrere Kohlenwasserstoff- (Lipid)-komponenten, ausgewählt aus unpolaren, insbesondere aliphatischen flüssigen, halbfesten und/oder festen Kohlenwasserstoffen (KW), ein oder mehrere polare Hilfsstoffe mit Emulgatoreigenschaften (EMU), und den Wirkstoff Permethrin (WS) in einer Menge von 8 bis 13 Gew. % umfasst, wobei das Verhältnis von Kohlenwasserstoffkomponente (KW) zu (WS) Wirkstoff Permethrin 1,5 bis 2,7 beträgt.

Die erfindungsgemäße Anwendung betrifft auch die Bekämpfung von Milben wie Krätzmilben bei Menschen, und dort betroffenen Arealen und insofern die Behandlung damit assoziierter Erkrankungen wie Krätze (Skabies).

## Beschreibung

### Gegenstand der Erfindung

Die vorliegende Erfindung betrifft eine halbfeste Creme-Zubereitung zur kutan = dermal-topischen Anwendung bei / Behandlung von i Ektoparasiten/Ektoparasiten-Befall, wobei die Creme-Zubereitung folgende Komponenten umfasst: Wasser, ein oder mehrere Kohlenwasserstoff- (Lipid)-komponenten, ausgewählt aus unpolaren, insbesondere aliphatischen flüssigen, halbfesten und/oder festen Kohlenwasserstoffen (KW), vor allem ein oder mehrere Vaseline, ein oder mehrere Paraffine oder Gemische hiervon, ein oder mehrere polare Hilfsstoffe mit Emulgatoreigenschaften (EMU), ausgewählt aus C₁₀ - C₂₈-Fettalkoholen, C₁₀ - C₂₈-Fettalkoholsulfaten, C₁₀ - C₂₈- Fettalkoholsulfatsalzen sowie Mischungen hiervon, und der Wirkstoff Permethrin (WS) in einer Menge von 8 bis 13 Gew. %, wobei das Verhältnis von Kohlenwasserstoffkomponente (KW) zu (WS) Wirkstoff Permethrin 1,5 bis 2,7, oder auch 1,9 bis 2,3 oder 1,8 bis 2,4, vor allem 2,0 bis 2,2, bezogen auf Gew.% von (KW) und (WS) in der Gesamtzubereitung, beträgt.

Die Zubereitung kann Zusatzstoffe in einer Menge von 0 bis 7 Gew. %, vor allem 0,01 bis 5 Gew. % aufweisen.

Die erfindungsgemäße Anwendung bezieht sich insbesondere auf die Bekämpfung von Milben, wie Krätzmilben bei Menschen, und dort betroffenen Arealen und insofern die Behandlung damit assoziierter Erkrankungen wie Krätze (Skabies).

Die erfindungsgemäße angewandte Zubereitung kann leicht kutan / dermal topisch auf die Haut / Anhangsgebilde von Säugetieren, insbesondere Menschen, aufgetragen werden. Sie weist überraschenderweise ein unerwartet hohes Penetrationsvermögen in die relevanten Hautschichten (mittleres bis unteres Stratum Corneum) sowie eine in Bezug auf die 2-fach aufgetragene, gemäß gegenwärtigem Standard konzentrierte (5%ige) Wirkstoffzubereitung eine höhere und damit überadditive Effektivität auf. Dies zeigte sich vor allem bei der vollständigen Beseitigung von Skabiesmilbenbefall (Heilung der Skabies) bereits zwei Wochen nach der ersten Anwendung.

Die Erfindung betrifft auch die Verwendung von Permethrin (WS) und einer oder mehreren unpolaren Kohlenwasserstoffen = Kohlenwasserstoff-(Lipid)-komponenten (KW), insbesondere von Paraffinen und / oder Vaselinen wie weißem Vaselin, gelbem Vaselin, dickflüssigem Paraffin, mikrokristallinem Paraffin, Hartparaffin (festes Paraffin), dünnflüssigem Paraffin oder Mischungen hiervon, im Gewichtsverhältnis (KW) zu (WS) von 1,5 bis 2,7, oder auch 1,9 bis 2,3 oder 1,8 bis 2,4, vor allem 2,2 bis 2,0, bezogen auf Gew.% von (KW) und (WS) in der Gesamtzubereitung, zur Herstellung einer halbfesten Cremezubereitung mit verbesserten Anwendungseigenschaften beim topisch-kutanen Einsatz gegen Ektoparasiten, vor allem Milben, insbesondere Krätzmilben und zur Behandlung der Krätze (Skabies). Die halbfeste Creme-Zubereitung umfasst, oder besteht aus 8 bis 13 Gew.% Permethrin (WS), 38 bis 73 Gew. % Wasser, 13 bis 34 Gew.% Kohlenwasserstoffkomponente (KW), 6 Gew.% bis 15 Gew.% eines oder mehrerer Hilfsstoffe mit Emulgiereigenschaften (EMU), vor allem ausgewählt aus C₁₀ - C₂₈- Fettalkoholen und Fettalkoholsulfaten, Fettalkoholsulfatsalzen, emulgierendem Cetylstearylalkohol Typ A oder Typ B sowie Mischungen hiervon, 0 bis 7%, vor allem 0,01 bis 5 Gew.% Zusatzstoffen, vor allem ausgewählt aus Konservierungsmitteln, Hautpflegestoffen, Antihistaminika, Kortikoiden oder Mischungen hiervon.

### Hintergrund der Erfindung

Halbfeste Zubereitungen zur kutanen (topischen) Anwendung umfassen im Allgemeinen eine geeignete Grundlage mit einem oder mehreren Wirkstoffen darin gelöst oder dispergiert. Die Grundlage kann ein- oder mehrphasig sein und je nach Inhaltsstoffen hydrophile oder hydrophobe Eigenschaften aufweisen. Der Begriff 'halbfest' bedeutet, dass die Zubereitungen in ihren Eigenschaften zwischen festen und flüssigen Zubereitungen stehen, vgl. ,Stephanie Jacobsen, "Arzneiformlehre: Ein Wegweiser in Bildern", 2 überarbeitete Auflage, 2013, ISBN 13: 978304731615, Kapitel 9`.

Zu den geeigneten Zubereitungen zur kutanen Anwendung zählen Cremes. Es handelt sich dabei um mehrphasige Systeme, insbesondere 2- phasig, aus Wasser- und lipohiler Phase. Bei Cremes kann man unterscheiden zwischen hydrophilen Cremes = Öl-in-Wasser- Cremes und lipophilen (hydrophoben) Cremes = Wasser-in-ÖI-Cremes, hydrophilen Salben oder Cremes, amphiphilen Cremes etc..

Von Bedeutung für die kutane Wirkung derartiger Zubereitungen sind u.a. der Aufbau und die Beschaffenheit der Haut. Diese wird im Wesentlichen in drei Schichten eingeteilt: die Epidermis, die Dermis (Cutis) und die Subcutis. Die Hautschichten weisen unterschiedliche Polaritäten und unterschiedliche Wassergehalte auf, siehe Jacobsen, a.a.O.

Wirkstoffe aus kutanen Zubereitungen können direkt durch die Zellen in die Haut eindringen (transzelluläre Route), oder auch durch Passage des Interzellularraums (interzelluläre Route).

Das Eindringen eines Wirkstoffes in die oberen Hautschichten (Epidermis) wird als Penetration bezeichnet, ein nachfolgendes Eindringen in tiefere Schichten (in den Bereich der Dermis) als Permeation. Das Eindringen in (tiefere) Hautschichten kann durch Hilfsstoffe verbessert werden (Penetrationsbeschleuniger, Enhancer), siehe auch Stephanie Jacobsen, "Arzneiformlehre: Ein Wegweiser in Bildern", 2 überarbeitete Auflage, 2013, ISBN 13: 978304731615, Kapitel 9`.

Eine Penetration bedingt eine lokale, im Grundsatz damit nicht systemische Wirkung, wohingegen bei relevanter Permeation eine relevante systemische Wirkung durch Eindringen in den Bereich der Blutgefäße der Haut nicht auszuschließen ist.

Eine kutane Anwendung kann bei unterschiedlichen Einflüssen auf die Haut erforderlich sein, z.B. zum Schutz gegen Austrocknung, Strahlung, bei Verletzungen, Wunden sowie Allergien, Ekzemen u.a., welche unterschiedliche Ursachen haben können.

Eine häufige Ursache sind Parasiten wie beispielsweise Ektoparasiten, die Menschen sowie deren Nutz- und Haustiere befallen können. Diese umfassen Anthropoden wie z. B. Zecken, Milben, Flöhe oder Läuse, Fliegen, Mosquitos und ähnliches und können zu ernsthaften Erkrankungen führen, sowohl durch direkte Wirkung (wie z.B. Hautläsionen, Ausschlag) als auch durch Übertragung pathogener Stoffe. Derartige Erkrankungen sind beispielsweise Enzephalitis, Borreliose, Malaria oder insbesondere Krätze (Fachbegriff: Skabies). Krätze ist eine durch spezielle Skabiesmilben verursachte ansteckende Krankheit, die durch engen Körperkontakt übertragen wird. Dabei graben sich die Milben in die oberste Hautschicht ein und legen dort Eier ab. Die Haut juckt, es bilden sich Ausschlag, Rötungen, Bläschen. Werden die juckenden Stellen aufgekratzt, bilden sich kleine Wunden. Wenn sich die Milben verbreiten, kann es zu einer zusätzlichen Infektion mit Bakterien kommen. Die Haut ist dann stark gerötet und nässt, manchmal bilden sich auch Eiter und Schorf.

Die durch die beschriebenen Parasiten verursachten gesundheitlichen Beeinträchtigungen, insbesondere auch die Krätze/Skabies bedürfen der Behandlung.

### Stand der Technik

EP 2816898 B1 beschreibt flüssige Kombinationsmittel aus 30 bis 55 Gew.% Permethrin, 2-15 Gew.% Fibronil mit einem speziellen Lösungsmittelsystem aus Myglyol und N-Methylpyrrolidon, um eine verbesserte Löslichkeit und Stabilität und damit verbesserte Effektivität der Wirkstoffe gegen Parasiten wie Milben zu erzielen.

EP 1379138 B1 (US7728011 B2) betrifft dermal applizierbare flüssige Formulierungen, enthaltend Permethrin und Agonisten oder Antagonisten der nicotinergen Acetylcholinrezeptoren von Insekten zur Bekämpfung von Flöhen und Zecken, umfassend 35-60 Gew.-% des Wirkstoffs Permethrin, 2.5-12.5 Gew.-% Imidacloprid oder Imidaclopridanalogon, 27.5-62.5
Gew.-% N-Methylpyrrolidon, 0-5 Gew.-% Wasser, 0-0,5 Gew.-% phenolische Antioxidantien und 0-0,5 Gew.-% organische Säuren.

Die Verwendung von topischen Formulierungen, enthaltend Permethrin (3-phenoxyphenyl) Methyl 3- (2, 2-dichloroethenyl)-2,2-dimethylcyclopropanecarboxylate (CAS No [52645-53-1] zur Bekämpfung von parasitierenden Insekten an Tieren ist bekannt, vgl. z. B. WO 95/17 090, betreffend eine nicht wässrige flüssige Zubereitung.

EP-A-0567368 betrifft flüssige wasserfreie Pestizidzusammensetzungen zur Bekämpfung von Insekten und Milben bei Tieren, welche Öle und aromatische Lösungsmittel aufweisen, insbesondere umfassend 200 bis 800 g/l Pflanzenöl und 300 bis 500 g/l Biphenylaromatisches Lösungsmittel und ein Pyrethroid, vor allem Deltamethrin oder Permethrin, wobei diese in Form von Bädern, Aerosolen oder nach dem sogenannten "pour on"-Verfahren angewendet werden sollen. Sie können auch in Shampoos, Lotionen oder Cremes verwendet werden, um beispielsweise Läuse beim Menschen zu bekämpfen. Eine beispielhafte Zubereitung ist zusammengesetzt aus (je g/L): Permethrin: 159, 6, BHT (Butylhydroxytoluol): 1, Calcium-Phenylsulfonat 30, Emulsogen EL 360: (PEG-36 Castor Oil) = ethoxliertes Rizinusöl: 20, Cyclohexanon: 150, Baumwollsamenöl 300, Biphenyl-Lösungsmittel 273. Als Biphenyl-aromatisches Lösungsmittel wird hier z. B. Dibenzyltoluol (Marlotherm SH, Gemisch aromatischer Kohlenwasserstoffe) genannt.

EP 0892606 B1 betrifft Formulierungen zur dermalen Bekämpfung von parasitären Insekten und Milben am Menschen mittels Agonisten oder Antagonisten der nicotinergen Acetylcholinrezeptoren von Insekten wie Chlornicotinyl-Insektizide, in einer Konzentration von 0,0001 bis 20 Gew.-%, Lösungsmittel aus der Gruppe der cyclischen Carbonate in einer Konzentration von 2,5 bis 99,9999 Gew.-%, gegebenenfalls weitere Lösungsmittel aus der Gruppe der Alkohole in einer Konzentration von 0 bis 95 Gew.-%.

EP 1587368 B1 beschreibt die Verwendung von antiparasitären Zubereitungen wie Permethrin (40/60 cis/trans) in Mengen von 15, vor allem 40 und mehr Gew.% zusammen mit nicotinergen Acetylcholinrezeptoragonisten in festen oder N-Methylpyrrolidon- haltigen flüssigen Zubereitungen zur Repellierung bei Anthropoden bei warmblütigen Tieren.

WO 93/24002 A offenbart, dass bestimmte 1-[N-(Halo-3-pyridyl-met-hyl)]- N-methylamino-1-alkylamino-2-nitroethylen-Derivate zur systemischen Anwendung gegen Flöhe bei Haustieren geeignet sind. Gemäß dieser WO 93/24002 ist die nichtsystemische - d.h. dermale - Applikationsweise für die Bekämpfung von Flöhen bei Haustieren ungeeignet.

EP0003251 A1 betrifft eine flüssige elektrostatisch sprühbare Insektizidzubereitung, umfassend eine Lösung aus 0,5 bis 50 % eines Insektizids wie Permethrin in einem organischen Lösungsmittel, wobei die Zubereitung einen Widerstand bei 20°C von 1 × 10⁶ bis 1 × 10¹⁰ Ohm Centimetres, und eine Viskosität bei 20°C im Bereich von 1 bis 50 Centistokes aufweist.

EP 4122468 A1 = WO2021/213483 A1 offenbart die Verwendung einer pharmazeutischen Zubereitung zur Kontrolle und Bekämpfung von Milben, insbesondere auch dadurch ausgelöster Augenerkrankungen wie Blepharitis marginalis, Meibom-Drüsen-Dysfunktion, Tarsitis, Madarosis, abnormaler Wimpernausrichtung, Glaukom und andere oder Hauterkrankungen bekämpft werden wie seborrhoische Dermatitis, Akne, Akne Rosacea, und dergleichen. Der Wirkstoff soll ausgewählt sein aus Taraxasterol, Taraxerol, Taraxeron, Roburinsäure, Taraxasterolacetat, Taraxerylacetat, Lupenon, Diosmetin oder Swertiamarin, Swerosid, Gentiopicrosid, Logansäure, insbesondere als ophthalmische Tropfen, Gel, Tinktur, Injektion, Salbe, Pulver, Aerosol.

EP 4129314 A1 /WO2021/213485 A1 betrifft pharmazeutische Zubereitungen zur Bekämpfung von Milben mit Löwenzahn- (Dandelion) Wirkstoffen, nämlich Monomerverbindungen gewählt aus Taraxerol, Diosmetin and Taraxasterolacetat. Die Zubereitung soll eingesetzt werden wie in EP 4122468 A1 geschildert.

US 6506396 B1 betrifft flüssige Shampoo-Formulierungen zur Anwendung bei Tieren, mit 0,1-10 % Agrikultur- / Pflege- oder Veterinärstoffen einschl. Insektiziden, b) 0,0002-40% eines Mikroemulsionskonzentrates, umfassend: (i) 0-10% Castoroilethoxylat oder Tristyrylphenolethoxylat, (ii) 0-1% ethoxylierte Phosphorsäure als pH Puffer, sowie N-C8-C18-Alkyl- Pyrrolidonen und N-C1-C4- Alkyl- Pyrrolidonen, Tensiden mit Shampoo Eigenschaften, und Wasser.

Für die Bekämpfung von Parasiten wie beispielsweise Milben lehrt der Stand der Technik somit die Anwendung verschiedener Wirkstoffkombinationen oder flüssiger, überwiegend nicht wässriger Permethrin-haltiger Produkte oder Kombinationsprodukte mit Permethrin, wobei organische Lösungsmittel, insbesondere aromatische Lösungsmittel wie N-Alkylpyrrolidone und / oder Biphenyllösungsmittel erforderlich sind, um eine geeignete Wirksamkeit zu erreichen.

Weiterhin lehrt der Stand der Technik gegen Milbenbefall die topische Anwendung eines halbfesten Produktes, umfassend 5 Gew. % Permethrin in Paraffin und Vaselin, Cetylstearylalkohol, emulgierender (Typ A), gereinigtes Wasser. Diese 5% -Zubereitung kann erhalten werden aus einem nicht zum Gebrauch geeigneten Rezepturkonzentrat aus 25 % Permethrin in Unguentum emulsificans aquosum DAB durch Verdünnen hiermit. Falls die Creme die Milben nicht abtötet, wie z. B. bei der ansteckenden Scabies crustosa, oder aus anderen Gründen nicht infrage kommt, ist es möglich, Tabletten einzunehmen oder andere Cremes zu verwenden, die andere Wirkstoffe enthalten.

Es gibt allerdings Hinweise aus der Literatur, dass die Wirksamkeit in solchen Formulierungen mit 5 Gew. % Permethrin in neuerer Zeit nachgelassen hat, so dass das entsprechende Produkt jetzt unter Umständen mehrfach oder häufiger angewendet werden muss. Es könnte sich dabei um eine Art Empfindlichkeitsreduktion der Skabiesmilben oder aber auch um neue Parasitenvarianten der Milben handeln.

### Aufgabe vorliegender Erfindung

Es besteht daher die Aufgabe vorliegender Erfindung, ein Mittel zum Einsatz gegen Ektoparasiten bereitzustellen, welches zum einen über eine gute Handhabbarkeit, Anwender- und Hautfreundlichkeit sowie eine gute Stabilität und darüber hinaus über eine verbesserte Effektivität verfügt. Insbesondere soll eine zuverlässige Wirksamkeit gegen Ektoparasiten, vor allem Milben, bei Lebewesen, wie vor allem Skabiesmilben beim Menschen, auf bzw. in der Haut, am Hals, Kopf, Beinen, Bauch, gesamten Körper, bei kürzest möglicher Anwendungszeit und möglichst wenig Behandlungswiederholungen, vor allem zur suffizienten Behandlung der Krätze/Skabies, gewährleistet werden.

### Lösung

Diese Aufgabe wird erfindungsgemäß gelöst durch die topisch dermale (äußere) Anwendung einer halb-festen, nicht flüssigen Creme-Zubereitung bei Ektoparasiten und zur Behandlung von durch diese hervorgerufenen Infestationen wie Skabies (Krätze) durch Milben umfassend, insbesondere bestehend aus, Wasser, eine unpolare Kohlenwasserstoff- (Lipid)-komponente (KW), vor allem ausgewählt aus einem oder mehreren Vaselinen, einem oder mehreren Paraffinen und Gemischen hiervon, einen(m) oder mehreren polaren Hilfsstoffen mit Emulgatoreigenschaften (EMU), ggf. Zusatzstoffen wie vor allem Konservieungsmittel sowie dem Wirkstoff (WS) Permethrin, eingesetzt als cis/trans Isomerengemisch mit cis- Anteil < trans- Anteil, in einer Menge von 8 Gew. % bis 13 Gew. %. Erfindungsgemäß beträgt das Verhältnis von Kohlenwasserstoffkomponente (KW) zu Wirkstoff (WS) 1,5 bis 2,7, oder auch 1,9 bis 2,3 oder 1,8 bis 2,4, insbesondere 2,0 bis 2,2.

Das Verhältnis von Kohlenwasserstoffkomponente(n) (KW) zu Permethrin- Wirkstoff (WS) ist bezogen auf die Gewichtsanteile der beiden Komponenten (Gew. % in der Gesamtzubereitung). Ganz besonders bevorzugt beträgt bei der erfindungsgemäßen Anwendung das Verhältnis (KW) zu (WS) 2,1.

Die Kohlenwasserstoffkomponente (KW) ist bevorzugt in einer Menge von 13 bis 34 Gew. %, vor allem 15 bis 26 Gew. %, vorhanden.

Erfindungsgemäß kann es auch vorteilhaft sein, wenn das Gewichtsverhältnis von emulgierendem Hilfsstoff (Hilfsstoff mit Emulgiereigenschaften) (EMU), vor allem emulgierenden Fettalkohol wie C₁₀ -C₂₈- Fettalkoholen und C₁₀-C₂₈- Fettalkoholsulfaten und deren Salzen, insbesondere Alkalisalzen, sowie Mischungen hiervon, und Permethrin-Wirkstoff (WS), 0,8 bis 1,3, vor allem 0,8 bis 1,0, insbesondere 0,9 beträgt, bezogen auf das Gewicht der Komponenten in der Gesamtzubereitung (Gew.%-Verhältnis).

Es können auch Zusatzstoffe, ausgewählt aus Konditioniermitteln, Hautpflegestoffen, Zusatzwirkstoffen wie Antihistaminika, Kortikoide, oder Mischungen derartiger Zusatzstoffe vorhanden sein. Konditioniermittel sind insbesondere Konservierungsmittel.

Der oder die Hilfsstoffe mit Emulgatoreigenschaften (EMU) sind vorzugsweise ausgewählt aus C₁₂-C₁₈- Fettalkoholen und C₁₂ -C₁₈- Fettalkoholsulfaten und / oder Sulfatsalzen, vor allem Natriumsalzen hiervon, sowie Mischungen hiervon, vor allem Laurylsulfat (Natriumsalz), Cetylalkohol, Stearylalkohol, Cetylstearylalkohol, Cetylstearylsulfat (Natriumsalz), insbesondere aus Emulgierendem Cetylstearylalkohol Typ A oder Typ B.

Der antiparasitäre Wirkstoff Permethrin (WS) umfasst 2 chirale Zentren und liegt als Isomerengemisch vor. Gemäß vorliegender Anmeldung werden alle cis:trans - Isomerengemische mit cis- Anteil < trans- Anteil, insbesondere cis-Anteil < 50%, bevorzugt < 40%, vor allem das 25:75 cis: trans- Isomerengemisch hiervon, umfasst und sind vor allem in einer Menge von 8 Gew. % -12 Gew.%, oder auch 9 Gew.% bis 11 Gew.%, insbesondere 10 Gew.% vorhanden. Ganz besonders bevorzugt wird das 25:75 cis:trans-Gemisch verwendet in einer Menge von 10 Gew. %.

Kohlenwasserstoff (Lipid)- Komponente (KW) bedeutet gemäß vorliegender Erfindung unpolare, insbesondere aliphatische flüssige und/oder halbfeste und / oder feste Kohlenwasserstoffe und vor allem derartige Gemische, welche vorzugsweise ausgewählt werden aus Paraffinen und Vaselinen, wie z. B. weißem Vaselin, gelbem Vaselin, mikrokristallinem Paraffin, Hartparaffin, dickflüssigem Paraffin, dünnflüssigem Paraffin (Paraffin Liquid und Paraffin Perliquid), oder Mischungen hiervon, insbesondere Mischungen aus Paraffin Liquid und weißem Vaselin und / oder Vaselin Flavium oder Paraffin Perliquid (dünnflüssig) und Vaselin, wie z.B. gelbes Vaselin, oder weißes Vaselin oder Vaselin Flavium oder Mischungen aus zweien oder 3 dieser Vaseline mit Paraffin Liquid oder Perliquid.

Ektoparasiten gemäß vorliegender Erfindung bedeuten Insekten oder Spinnentiere, deren Entwicklung ganz oder teilweise auf oder in der Haut eines Wirts erfolgt, beziehungsweise die für ihre Entwicklung Blutmahlzeiten, Zell- oder Lymphflüssigkeiten oder auch Hautbestandteile des Wirtes benötigen. Sie umfassen vor allem Zecken und Milben sowie Flöhe, Läuse, Haarlinge, Stechmücken, Fliegen und Sandmücken.

Im Sinne vorliegender Erfindung sind Milben winzig kleine Spinnentiere (Arachnida). Sie können Tiere (z.B. Hund, Katze) und auch Menschen befallen. Spezielle Arten von Milben sind beispielsweise Demodex-Milben, oder Krätzmilben (Sarcoptes scabiei, insbesondere auch die human-spezifischen Sarcoptes scabiei var. hominis). Weibliche Krätz- = Skabiesmilben werden 0,3 bis 0,5 mm groß, männliche Milben 0,21 bis 0,29 mm. Der Befall durch Sarcoptes scabiei verursacht beim Menschen die Skabies/Krätze mit den entsprechenden Hauterscheinungen und oft starkem, vor allem nächtlichem Juckreiz.

Die erfindungsgemäße Anwendung betrifft vor allem Säugetiere. Säugetiere gemäß vorliegender Erfindung bedeutet Haustiere (wie z.B. Hunde), Nutztiere (wie Rind, Schaf, Ziege oder auch Pferd und Schwein), und insbesondere den Menschen.

Im Sinne vorliegender Erfindung bedeutet halbfest eine nicht flüssige, nicht feste Zubereitung wie von Jacobsen oben erläutert, welche im Ruhezustand in festen Behältern wie Tuben, Kruken abgefüllt und auf Druck, Schub oder Ähnliches formbar appliziert werden kann. Damit handelt es sich nicht um eine Emulsion, welche flüssig ist (und nicht wie erfindungsgemäß halbfest) und ein höheres Fließverhalten hat als eine halbfeste Zubereitung.

Topisch kutan= dermale (äußerliche) Anwendung bedeutet gemäß vorliegender Erfindung, dass die Zubereitung geeignet ist zur Auftragung auf ein vorgesehenes kleineres oder größeres Hautareal, insbesondere auch auf dem ganzen Körper, je nach Bedarf. Die Menge hierbei kann z.B. 25 g bis 60 g pro Ganzkörperanwendung sein (je nach Körpergewicht und Größe).

Penetration im Sinne vorliegender Erfindung bedeutet wie oben erläutert das Eindringen eines Wirkstoffs in eine Hautschicht, hier insbesondere das untere und mittlere Stratum Corneum, nicht jedoch das relevante Durchdringen in den Bereich der Dermis bzw. Blutgefäße.

### Anwendungseffektivität

Überraschenderweise kann eine höhere Menge Permethrin als bisher (5 Gew.%), nämlich 8 bis 13 Gew. %, oder 9 bis 12 Gew. %, insbesondere 10 Gew. %, jeweils bezogen auf das Gesamtgewicht der Zubereitung, erfindungsgemäß mit einer Anwendung eingesetzt werden. Es sind im Übrigen auch keine höheren Mengen an organischen Lösungsmitteln hierzu erforderlich und auch keine wasserfreien Zubereitungen (also maximal bis zu 5 Gew. % Wasser), um noch eine geeignete Penetration und Wirksamkeit zu gewährleisten.

Dabei hat sich gezeigt, dass bei Verwendung des Wirkstoffs Permethrin (WS) und flüssigen, festen und / oder halbfesten vor allem aliphatischen Kohlenwasserstoffen (KW) bzw. Gemischen hiervon, insbesondere wie vorstehend genannt (Paraffine, Vaseline), im Gewichtsverhältnis (KW) zu (WS) von 1,5 bis 2,7, oder auch 1,9 bis 2,3 oder 1,8 bis 2,4, vor allem 2,0 bis 2,1, halbfeste Creme-Zubereitungen herstellbar und einsetzbar sind mit unerwartet hoher, überadditiver Wirksamkeit bei der Bekämpfung / Behandlung von Ektoparasiten-Befall wie vor allem Milben/Krätze.

Die erfindungsgemäß verwendete halbfeste Zubereitung ist stabil und wird topisch auf die Haut, also kutan appliziert. Sie wirkt dermal (nicht systemisch) in den oberen Hautschichten, hier insbesondere im unteren und mittleren Stratum Corneum, wo eine erhöhte Wirkstoffkonzentration erreicht wird. Dabei kann ein verbessertes Penetrationsprofil gegenüber dem Stand der Technik erreicht werden. So führt die erhöhte Wirkstoffverfügbarkeit in den relevanten Hautschichten unerwarteter Weise zu einer mehr als additiven Effekt im Vergleich zu der gleichen angewendeten Menge aus hälftig konzentrierten Standard-Produkten (wie bisher bekannt und oben beschriebene 5% haltige Permethrin-Cremes). Dies zeigte sich anhand von klinischen Untersuchungen im Zusammenhang mit Milben, in der ein höherer Therapieerfolg als es zu erwarten gewesen wäre, aufgezeigt werden konnte: bei Patienten mit dermatologisch gesicherter Skabies und Versagen einer 2fachen Permethrin-Therapie ergab sich nach doppeltem Auftrag einer im Vergleich zur erfindungsgemäß (angewendeten) Creme halbdosierten Standard-Creme, nämlich 2x 5% Permethrin, binnen 24 h bei Kontrolle nach zwei Wochen lediglich ein Therapieerfolg von 11%, während bei Einmalauftrag der erfindungsgemäßen 10% Permethrin Creme (1x 10%) ein Therapieerfolg (Heilung der Skabies / vollständige Eradikation aller Skabiesmilben) von 33% zu verzeichnen war (siehe nachfolgende Tabelle 2). Das ist überraschend, da bei Auftragung der gleichen Permethrin-Gesamtmenge durch die erfindungsgemäße 10% Permethrin Creme binnen 24 h ein dreifach erhöhter Effekt erreicht werden kann und dies mit nur einer Anwendung. Zusätzlich wurde bei o.g. Untersuchung nach einer 2. Anwendung von 10% Permethrin Creme bei den nach 14 Tagen noch nicht geheilten Patienten ein Therapieerfolg von insgesamt sogar 50 % gefunden. Die erfindungsgemäße Anwendung führt überraschend sogar bei vorheriger Nichtwirksamkeit von 5%iger Permethrin Creme bei jedem 2. Patienten doch noch zu einer Heilung durch eine besser geeignete, eben die erfindungsgemäße Permethrin-Creme-Zubereitung.

Die erfindungsgemäß eingesetzte halbfeste Zubereitung zur dermal topischen Verwendung bei Ektoparasiten, insbesondere Milbenbefall, Krätze, kann somit eine in Bezug auf eine halbkonzentrierte, doppelt aufgetragene Zubereitung gemäß Standard - 5% Permethrin-Creme (also 2x 5% Gew.% Wirkstoffkonzentration gegenüber 1x 10 Gew.%) einen überadditiven Effekt bei der Beseitigung von Milbenbefall, respektive der Behandlung von Krätze aufweisen.

### Nähere Erläuterung der Erfindung und der einzelnen Komponenten der Zubereitung

Die erfindungsgemäß verwendete halbfeste Zubereitung zur kutanen (dermalen) Anwendung ist insbesondere eine hydrophile Creme und umfasst neben Wasser ein oder mehrere unpolare Kohlenwasserstoff- (Lipid)-Komponenten (KW), ein oder mehrere Hilfsstoffe mit Emulgiereigenschaften (EMU) und ggf. Zusatzstoffe.

Die Zubereitung weist weiterhin den Wirkstoff Permethrin (WS) auf, der als antiparasitärer Wirkstoff in der Zubereitung vorliegt.

Weitere antiparasitäre Wirkstoffe können, falls gewünscht, vorhanden sein, sind bevorzugt aber nicht enthalten.

### 1. Wirkstoff (WS) Permethrin

Permethrin gemäß vorliegender Erfindung ist ein Insektizid und Akarizid aus der Gruppe der Pyrethroide. Es wirkt als Kontakt- und Fraßgift; sein Wirkungsspektrum ist sehr breit. Permethrin ist seit etwa 1977 im Handel. Es ist ein Typ-I-Pyrethroid, hat also keine Cyano-3-phen-oxybenzyl-Gruppe.

Die chemische Bezeichnung ist:
3-(2,2-Dichlorethenyl)-2,2-dimethylcyclopropancarboxylsäure-(3-phenoxyphenyl)methylester

Oder 3-(Phenoxy-1-yl)benzyl 3-(2,2-dichlorethen-1-yl)-2,2-dimethylcyclopropancarboxylat (siehe https://de.wikipedia.org/wiki/permethrin).

Der chemischen Formel ist:

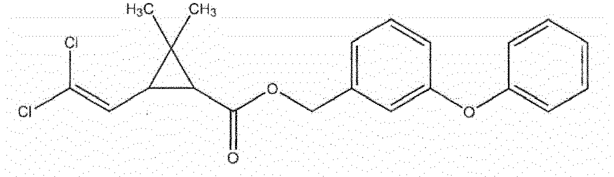

Permethrin ist bei RT fest (Schmelzpunkt ca. 34-35°C) und nahezu unlöslich in Wasser, (0,2 mg·l⁻¹ bei 20 °C).

Permethrin hat aufgrund der zwei Stereozentren im Cyclopropan-Ring vier Stereoisomere (zwei Enantiomerenpaare). Das trans-Enantiomerenpaar ist als Transpermethrin bekannt. siehe https://de.wikipedia.org/wiki/Permethrin.

Vorzugsweise verwendet wird das Enantiomerenpaar cis / trans 25:75. Geeignet können auch andere cis / trans Verhältnisse sein, wenn der cis-Anteil jeweils geringer ist als der trans-Anteil.

### 2. Wasser

Die Menge an Wasser, insbesondere gereinigtem Wasser, beträgt vor allem 38 Gew. % bis 73 Gew.%, insbesondere bis 42 Gew. % bis 65 Gew. %, oder auch 30 bis 65 Gew. %, vor allem 45 Gew.% bis 65 Gew. %, insbesondere 60 Gew. %, jeweils bezogen auf die Gesamtmenge der Zubereitung.

### 3. Kohlenwasserstoff (=Lipide) Komponente (KW)

Unpolare Kohlenwasserstoffe (KW) bilden die Lipidkomponente. Je nach Kettenlänge sind diese Kohlenwasserstoffe fest, halbfest, flüssig oder Mischungen hiervon. Die Gew. %-Angaben (13 Gew. % bis 34 Gew. %, bevorzugt 15 Gew. % bis 26 Gew.%, insbesondere 17 Gew.% bis 22 Gew.%) sind dabei bezogen auf die Summe aller enthaltenen Kohlenwasserstoffe in der Gesamtzubereitung.

Zu diesen für die erfindungsgemäß verwendete Creme geeigneten Kohlenwasserstoffen (KW) gehören insbesondere Vaseline sowie Paraffine. Hierbei handelt es sich um vorwiegend aliphatische Kohlenwasserstoffe, welche je nach Kettenlänge und Verzweigungsgrad flüssige bis halbfeste oder feste Kohlenwasserstoffe aus Petroleum darstellen, mit überwiegend aliphatischen Anteilen. Dem Fachmann sind diese Produkte bekannt. Sie sind auch in pharmazeutischen Qualitäten erhältlich.

Derartige Paraffine und Vaseline werden nachfolgend beispielhaft beschrieben:
Vaselin (= Petrolatum) ist allgemein bekannt als unpolares halbfestes KohlenwasserstoffGemisch. Dabei können 70 bis 90 % aus einem flüssigen Anteil aus stark verzweigten iso-Paraffinen und Olefinen, die einen Schmelzpunkt von 35 °C bis 60 °C aufweisen und ein fester (kristalliner) Anteil (10-30 %) langkettiger Komponenten (n-Paraffine und wenig verzweigte iso-Paraffine) vorliegen, siehe https://www.chemie.de/lexikon/Vaseline.

Je nach Anteil aromatischer Kohlenwasserstoffe liegt Vaselin als gelbes oder weißes Vaseline vor.

Die Kettenlänge der Kohlenwasserstoffe hierbei kann C₁₅-C₆₀, der mittlere C-Bereich C₂₆, sein, oder es können auch Kohlenwasserstoffe mit C-Anteil > 20 vorliegen.

Es kann auch ein Gemisch aus C₂₄-C₃₄ Kohlenwasserstoffen umfasst sein (wie in EP2550954 beschrieben), insbesondere im weißen Vaselin.

Pharmazeutische Qualitäten (gemäß Europäischem Arzneibuch Ph.Eur.) des weißen und gelben Vaselins (lateinisch Vaselinum album und Vaselinum flavum, auch bekannt als Paraffin white soft bzw. yellow soft) unterscheiden sich neben dem Aussehen dahingehend, dass im weißen Vaselin weniger unerwünschte aromatische, polycyclische Kohlenwasserstoffe ("MOAH") vorhanden sein sollen als bei gelbem Vaselin.

Der Tropfpunkt (Übergang fest-flüssig) für weißes Vaselin kann bei 35° C-75 °C, bei gelbem Vaselin bei 40-60 °C (https://de.wikipedia.org/wiki/Vaseline) oder auch oder auch bei 35° bis 70° C (Ph. Eur) liegen.

In den erfindungsgemäß eingesetzten Zubereitungen wird bevorzugt weißes Vaselin wie Vaseline white = Vaselinum album = Paraffin white soft = halbfeste Kohlenwasserstoffmischung aus Petroleum, z. B. mit Tropfpunkt 35°C bis 75° C oder auch 35°C bis 70° verwendet.

Paraffine sind Gemische aus acyclischen Alkanen (gesättigten Kohlenwasserstoffen), die molare Masse kann beispielsweise zwischen 275 und 600 g/Mol betragen. Sie können als feste Massen, halbfest oder flüssig vorliegen.

Feste bis halbfeste Vertreter hierzu sind z.B. Hartparaffin, auch mikrokristallines Wachs, mit Schmelzpunkt zwischen 50° C und 62 °C, oder Weichparaffin mit Schmelzpunkt bei ca. 45 °C.

Mikrokristalline Paraffine (Wachse) sind gemäß Ph.Eur. gereinigte Gemische fester geradkettiger, verzweigter und zyklischer gesättigter Kohlenwasserstoffe mit Topfpunkt > 65° C. Mikrokristalline Wachse können Erstarrungspunkte zwischen 70 °C und 80 °C aufweisen und Kettenlängen von bis zu 75 Kohlenstoffatomen enthalten (siehe https:// www.chemie.de/-lexikon/Paraffin).

Bei flüssigen Paraffinen, die eine Dichte von 0,81-0,89 g/cm aufweisen können), kann je nach Kettenlänge und Verzweigungsgrad der Kohlenwasserstoffe allgemein unterschieden werden zwischen dünnflüssigen Paraffinen (Paraffinum perliquidum), z. B. mit einer Viskosität von 25 bis 80 mPas, und dickflüssigen Paraffinen (Paraffinum subliquidum), die als ölige Flüssigkeit vorliegen ( z.B. mi einer Viskosität von 110 bis 230 mPas).

Dementsprechend variieren die Viskositäten derartiger Paraffinprodukte (Wachs: Schmelzpunkt 50-62 °C, Paraffinum Light: Viskosität > 100cp, 20 °C, medizinisches Mineralöl: 65 cp/20 °C).

### Beispiele hierfür sind:

Dünnflüssiges Paraffin (Paraffinum perliquidum, Paraffin light liquid (Ph.Eur. mit Dichte 0,810-0,875), Paraffinum flüssig (Paraffinum Liquid, z. B. gemäß Ph.Eur: Paraffinum Liquid, Dichte: 0,827-0,890) oder Dickflüssiges Paraffin (Paraffinum subliquidum).

Bekannt sind derartige Paraffin- Produkte auch als Paraffin, Paraffinum white (= mikrokristallines Wachs), Paraffinum light (leichtes Mineralöl).

Die Kohlenwasserstoffe und Mischungen hiervon wie oben erläutert können wie erläutert unterschiedliche Zusammensetzungen entsprechend der Kettenlängen und Verzweigungen der Komponenten und die allgemeine, nicht ausschließliche Formel Cₙ-H₂ₙ₊₂ mit n = 16-75, vor allem CₙH₂ₙ₊₂ mit n = 18-60 vor allem 18-36, (z.B. für Paraffine), aufweisen. Es handelt sich hauptsächlich um unpolare aliphatische acyclische gesättigte, ggf. ungesättigte, geradkettige, flüssige, halbfeste oder feste Kohlenwasserstoffe oder Mischungen sowie aliphatische gesättigte verzweigte (iso-) Kohlenwasserstoffe, ggf. mit geringen Anteilen an ungesättigten Verbindungen oder auch aromatischen Verbindungen, deren Menge vom Reinheitsgrad abhängen kann.

Die in erfindungsgemäßen Zubereitungen eingesetzten Vaselin- bzw. Paraffin-Produkte sind gleichermaßen verwendbar. Besonders bevorzugt werden Kohlenwasserstoff-Gemische (KW) wie Vaseline (Petrolatum), z. B. wie oben beschrieben, aus 70 bis 90 % eines flüssigen Anteils aus stark verzweigten iso-Paraffinen und Olefinen und einem festem kristallinen Anteil (10-30 %) aus langkettigen Komponenten (n-Paraffine und wenig verzweigte iso-Paraffine), vor allem in einer (Gesamt)Menge von 20 bis 30 Gew.%.

Weiterhin bevorzugt sind Paraffine wie Paraffin Liquid (gereinigte Mischung aus flüssigen Kohlenwasserstoffen), Light Paraffin oder Paraffinum Perliquid (flüssiges oder dünnflüssiges Paraffin), medizinisches (flüssiges) Paraffin, Paraffinum subliquidum (dickflüssiges Paraffin) oder Mischungen hiervon, vor allem in Gesamt-Mengen von 18 bis 30 Gew.%, vor allem 20 bis 25 Gew.%.

Anmeldungsgemäß besonders geeignete Mischungen von Kohlenwasserstoffen (KW) sind Vaselin + dickflüssiges Paraffin (Paraffinum subliquidum), Vaselin weiß, Vaselinum album und/oder Paraffinum white soft + dickflüssiges Paraffin (Paraffinum subliquidum).

### 4. Hilfsstoffe

Als Hilfsstoffe werden insbesondere solche mit emulgierenden (Emulgator)-Eigenschaften gewählt. Diese werden eingesetzt in Mengen von 6 Gew. % bis 15 Gew. %, bevorzugt 7 Gew. % bis 11 Gew. % und ganz besonders 9 Gew. %, bezogen auf die Gesamtzubereitung.

Zu diesen Hilfsstoffen gehören vor allem C₆-C₃₀, vor allem C₁₀-C₂₈-aliphatische, einwertige Fettalkohole, wie sie in Wachsen (gebunden in Estern) vorkommen, (daher auch Wachsalkohole genannt), insbesondere im Gemisch mit ionischen, vor allem anionischen Sulfatsalzen hiervon, vor allem Sulfatalkalisalzen hiervon (Tensidsulfate aus Fettalkoholen). Die Salze, insbesondere Sulfatsalze oder Alkalisulfatsalze der Fettalkohole können als ionische, vor allem anionische (emulgierende) Hilfskomponenten eingesetzt werden.

Zu den aliphatischen Fettalkoholen gehören z.B. die gesättigten Vertreter wie 1- Decanol (C10), Dodecanol = Laurylalkohol (C₁₂), Laurylalkohol, Myristylalkohol (C₁₄), Cetylalkohol (C₁₆), Stearylalkohol (C₁₈), 1 Docosanol (C₂₂), Ligocerylalkohol C₂₄)oder auch einfach ungesättigte Verbindungen wie cis-9-Octadecen-1-ol (Oleylalkohol (C₁₈), oder Linoeylalkohol Octradecatrien-1-ol (C₁₈,3-fach ungesättigt) oder Sulfate hiervor wie Natriumlaurylsulfat, Cetylsulfat, (Natrium) Natriumstearylsulfat, Natriumcetylstearylsulfat.

Insbesondere geeignet sind Produkte, die unter dem Namen, Lanette-Wachse' bekannt sind. Es handelt sich dabei um Cetylstearylalkohol. Letzterer ist ein Gemisch fester aliphatischer Alkohole, hauptsächlich Cetylalkohol (Hexadecan-1-ol) und Stearylalkohol (Octade-can-1-ol), tierischen oder pflanzlichen Ursprungs.

Cetylstearylalkohol ist eine wachsartige Masse von weißer bis blassgelber Farbe. Pharmazeutische Qualitäten enthalten mindestens 40,0 Prozent Stearylalkohol, die Summe der Gehalte von Stearylalkohol und Cetylalkohol beträgt mindestens 90,0 Prozent. Die Schmelztemperatur liegt bei 49 °C bis 56 °C. Cetylstearylalkohol hat einen HLB-Wert von circa 1 und wirkt nur schwach grenzflächenaktiv (W/O-Emulgator).

Derartige Fettalkohole können mit anionischen Komponenten kombiniert werden: emulgierender Cetylstearylalkohol ist ein (pharmazeutisch verwendbarer) Mischemulgator, der aus mindestens 80,0 Prozent Cetylstearylalkohol und 7,0 Prozent der ionischen O/W-Emulgatoren Natriumcetylstearylsulfat (Typ A) oder Natriumlaurylsulfat (Typ B) besteht. Sowohl emulgierender Cetylstearylalkohol vom Typ A als auch vom Typ B sind löslich in heißem Wasser unter Bildung einer opaleszierenden Lösung, jedoch praktisch unlöslich in kaltem Wasser. Mit diesen Komponenten können hydrophile Grundlagen wie "Anionische hydrophile Cremes DAB" bereitet werden.

Als Handelsprodukte sind Lanette O (Cetylstearylalkohol), Lanette E (Natriumcetylstearylsulfat) bzw. Lanette N (Emulgierender Cetylstearylalkohol (Typ A: Cetearyl Alcohol und Natriumcetearylsulfate), Lanette SX (emulgierender Cetylstearylalkohol Typ B: Cetearyl alkohol + Natriumlaurylsulfat + Natriumcetearylsulfat) bekannt, siehe auch https://de.wikipedia.org/wiki/Cetylstearylalkohol.

Besonders bevorzugt wird erfindungsgemäß Cetylstearylalkohol Typ A oder Cetylstearylalkohol Typ B, jeweils in einer Menge (Gew.%) von 6-15, insbesondere 7 Gew.% bis 11 Gew.%, vor allem 8 Gew.% -10 Gew.%, insbesondere 9 Gew.% eingesetzt.

### 5. Zusatzstoffe

Zusatzstoffe können vorzugsweise gewählt werden aus Hautpflegestoffen, Konditioniermitteln, Zusatzwirkstoffen oder Mischungen hiervon. Sie liegen in Mengen von 0 bis 7 Gew. %, vorzugsweise 0 bis 5%, vor allem 0,01 bis 5 Gew. %, insbesondere 0,01 bis 3 Gew. %, bezogen auf das Gewicht der Gesamtzusammensetzung, vor.

### 5.1. Hautpflegestoffe

Zu geeigneten Hautpflegestoffen, insbesondere zur Feuchtigkeitsregulation und zum Schutz gegen äußere unerwünschte Einflüsse, gehören vor allem lipophile polare Komponenten aus der Gruppe der Fette, fetten Öle. Im Sinne vorliegender Erfindung handelt es sich hierbei nicht um unpolare Lipidkomponenten (KW) und auch nicht um Hilfsstoffe mit Emulgatoreigenschaften wie erfindungsgemäß definiert (welche Fettalkohole und deren Salze / -Sulfatsalze umfassen).

Bei den lipophilen polaren Hautpflegestoffen handelt sich vor allem um Glycerid-Ester (Mono-,Di-Triglyceride) von gesättigten, ungesättigten oder mehrfach ungesättigten C₁₂-C₂₄-Fettsäuren, gesättigten, ungesättigten oder mehrfach ungesättigten C₁₂-C₂₄ Fettsäureglykolester oder C₁₂-C₂₄ Fettsäure- C₂-C₁₈ Alkohol-Ether sowie um Wachsester (C₁₂-C₂₄ Fettsäureester von aliphatischen C₆- bis C₂₂Alkoholen). Zu den Glyceridestern gehören natürliche Öle wie Sojaöl, Kokosnussöl, Jojobaöl, Avocadoöl, Erdnussöl, Traubenkernöl, Sonnenblumenöl, Borretschöl, Sesamöl, Olivenöl, Aprikosenkernöl, Maiskeimöl, Weizenkeimöl, Walnussöl, Palmöl, Macadamianussöl, Palmkernöl, Haselnussöl, Hagebuttenkernöl, Baumwollsamenöl, Mandelöl, Mandelbutter, Lecithin, Distelöl, Macademiaöl, Papayaöl. Bei diesen Ölen handelt es sich in der Regel um Triglyceride von gesättigten, ungesättigten oder mehrfach ungesättigten C₁₂- C₂₄-, insbesondere C₁₂-C₁₈-Fettsäuren oder Mischungen hiervon wie Linol-/Linolen-Säure, Palmitinsäure, Ölsäure oder auch übliche Lipide wie Fettsäureester wie mittelkettige (z.B. C₈₋₁₂) Triglyceride. Geeignet sind auch Mono- und Diglyceride wie Monoglycerolstearat, Diglycerolstearat.

Weiterhin gehören zu dieser Gruppe mittelkettige Propylenglyokolester wie z. B. Fettsäureester, z.B. C₂₋₁₈-Alkoholfettsäureester wie Isopropylfettsäureester (Palmitat, Myristat, Isostearat, Oleat), Decyl Oleate, Hexyl Laurate, C₁₂-C₁₅-Alkyl Benzoate, Dicaprylyl-Carbonate sowie verzweigte Fettsäureester wie Cetearyl Octanoate, Di-n-Butyladipate oder auch C₈₋₂₂- Fettalkoholether wie Dicaprylyl Ether.

Zu den Wachsen gehören natürliche und synthetische Wachse. Natürliche Wachse sind Ester von Fettsäuren wie oben erläutert (auch Wachssäuren genannt) mit langkettigen, aliphatischen, primären Alkoholen, den so genannten C₆- bis C₂₂-, insbesondere C_{10'}-C₁₈-Fettalkoholen (wie beispielsweise Cetylalkohol, Stearylalkohol und/oder Cetylstearylalkohol).

Tierische Wachse sind beispielsweise Wollwachs (Lanolin= Ester von langkettigen Hydroxysäuren und Hydroxyalkoholen und auch Cholesterol, Lanosterol, Agnosterol), Chinawachs (Ester von C24-C32- Fettsäuren und C24-C32- Fettalkoholen), Bienenwachs und Bürzeldrüsenfett.

Zu den pflanzlichen Wachsen gehören Zuckerrohrwachs, das Carnaubawachs der Carnauba-Wachspalme, Candelillawachs. Weitere pflanzliche Wachse sind Korkwachs, Guarumawachs (Calathea lutea), Ouricuriwachs (Syagrus coronata), Kuba-Palmenwachs (Copernicia hospita), Espartowachs (Lygeum spartum, Stipa tenacissima), Baumwollwachs, Reiskleiewachs, Flachswachs, Torfwachs und Rosenwachs, Jasminwachs.

Synthetische und halbsynthetische Wachse sind beispielsweise flüssige Wachse wie synthetische Fettsäureester, z. B. Myristinsäureisopropylester, Isopropyloleat, Oleyloleat u. a., rekonstruierte Wachse, z. B. Cetylpalmitat.

Weitere Feuchthalter sind z.B. Glycerin, Polyalkohole wie Polyethylenglykol, Propylenglykol, Butylenglykol, Sorbitol, Glycerol oder Polymere, z.B. Polyquaternium-Typen wie Polyquaternium -39 (Merquat^{®} plus 3330), Proteine wie Collagen oder dessen Hydrolysate, Aminosäuren, Harnstoff, D-Panthenol, pflanzliche Proteine wie z.B. aus Weizen, Soja oder Mandel oder deren Hydrolysate.

Auch können Vitamine als Hautpflegestoffe verwendet werden. Hierzu gehören z.B. Vitamin C, Thiamin, Riboflavin, Niazin, Pantothensäure, Pyrridoxin, Folsäure, Kobalamin, Orotsäure, para-Aminobenzoesäure, Inosit, Carnitin, Bioflavonoide, Ascorbinsäure, Biotin, Alfa-Toco-pherol, Retinol und Derivate, Beta-Karotin, Liponsäure.

### 5.2. Konditioniermittel

Hierzu gehören Konservierungsmittel wie vor allem Sorbinsäure und/ oder Sorbate wie Kaliumsorbat, oder auch Benzylalkohol, Salicylsäure, Phenoxyethanol, Parabene (wie Ethyl,-Methyl-, Propylparaben usw.), Dehydracetsäure und deren Salze, Methyldibromoglutanonitril, etc. oder Kombinationen hiervon, oder Ester wie p-Hydroxy-Benzoesäureester.

Weitere Stabilisatoren können Antioxidantien sein wie Butylhydroxytoluol, Butylhydroxyanisol, Ascorbylpalmitat, Carotin, Vitamin A, Tocopherol, Gallussäurealkylester wie Octyl-, Dodecyl- und Cetylgallat oder Kombinationen hiervon.

Gegebenenfalls können auch Komponenten mit farbgebenden Eigenschaften zur Konditionierung beitragen.

Weitere Konditioniermittel können pH Wert Regulatoren sein, wie NaOH, oder Säuren wie z.B. Zitronensäure, Milchsäure oder Äpfelsäure, Puffer oder EDTA.

Andere bekannte Konditioniermittel sind beispielsweise (jeweils in bekannten geeigneten Mengen) Stärke, Salze wie NaCl, Polysaccharide (z. B. Xanthan Gum), Cellulosederivate (z.B. Methylcellulose, Hydroxyethylcellulose, Carmellose-Na, Hydroxypropylcellulose, Hypromellose) oder Stärkeglycola, Reis-, Weizen-, Mais- und Kartoffelstärke, oder Silikate wie Magnesium Aluminium Silikat und ähnliches, welche auch zur Viskositäts-und/oder Konsistenzregulation (Konditionierung) beitragen.

### 5.3 Zusatzwirkstoffe

Unter bestimmten Anforderungen können auch Zusatzwirkstoffe inkorporiert sein in die erfindungsgemäß verwendete Zubereitung wie ätherische Öle und/oder Pflanzenextrakte (welche auch als Duftstoffe vorhanden sein können, z.B. Öle/Extrakte aus Ylang Ylang, Kiefernnadel, Zypresse, Thymian, Minze, Limetten, Orangen, Grapefruit, Mandarine, Wacholder, Baldrian, Zitronenmelisse, Eukalyptus, Thymian, Palmarosa, Rosmarin, Lavendel, Rosenholz, Lemongras, Fichtennadel, Kiefernnadel, Ingwer-, Johannisbeer-, Lindenblüten-, Ringelblumen-, Magnolien-, Algen-, Aloe Vera-, Ananas-, Guave-, Echinacea-, Efeublättextrakt, Birkenblätterextrakt, Ringelblumen-Hibiskus-, Klettenwurzel, Hamamelis-, Wassernabelkraut-, Quitten-, Wasserlilien-, Zimtextrakt oder Mischungen hiervon.

Als Zusatzwirkstoffe können vor allem auch Antihistaminika und / oder Kortikoide vorhanden sein in der erfindungsungemäß verwendeten Zubereitung.

Zu den Antihistaminika gehören vor allem H₁, H₂- oder ähnliche Rezeptor-Antagonisten wie Clemastin, Dimetinden,Diphenhydramin, Doxylamin, Meclozin bzw. Azelastin, Bilastin, Cetirizin, Desloratadin, Ebastin, Fexofenadin, Levocabastin, Levocetirizin, Loratadin, Terfenadin, oder Cimetidin, Famotidin, Lafutidin, Nizatidin, Ranitidin , Roxatidin, Thioperamid.

Zur topischen Anwendung sind vor allem folgende Antihistaminika bekannt:
Thonzylamin, Tripelennamin, Chloropyramin, Promethazin, Tolpropamin, Dimetinden, Clemastin, Bamipin, Pheniramin, Isothipendyl, Diphenhydramin, Diphenhydraminmethylbromid, Chlorphenoxamin, Diphenylpyralin, Dioxopromethazin, Diphenhydramin, Histapyrrodin, oder auch die in der Augenheilkunde eingesetzten Substanzen Antazolin (Spersallerg^{®}), Azelastin (Allergodil^{®}), Bilastin (Bilaxten^{®}), Emedastin, (Emadine^{®}), Epinastin (Relestat^{®}), Levocabastin-Augentropfen (Livostin^{®}) bzw. deren pharmazeutisch verträglichen Salze. (Siehe auch https://de.wikipedia.org/wiki/Antihistaminikum, https://www.gelbe-liste.de/atc /Antihistaminika-zur-topischen-Anwendung_D04AA).

Zu geeigneten Kortikoiden gehören insbesondere topische Glucocorticoide (Dermokortikoide), vor allem Verbindungen, die von körpereigenen Glucocorticoiden abgeleitet und ein Steroidgrundgerüst und unterschiedliche Wirkungsstärken aufweisen. Hierzu gehören z.B. Fluticasonpropionat, Fluticasonfuroat Hydrocortisonacetat (Dermacalm-d^{®}, Sanadermil^{®}), Prednisolonacetat (z.B. Imacort^{®}), Clobetasonbutyrat (Emovate^{®}), Hydrocortisonbutyrat (Locoid^{®}), Fluprednidenacetat (Decoderm^{®} bivalent), Triamcinolonacetonid (z.B. Pevisone^{®}), Dexamethasonacetat (z.B. Doxiproct^{®} Plus), Betamethasonvalerat (z.B. Betnovate^{®}), Fluocinolonacetonid (Synalar^{®}), Diflucortolonvalerat (Travocort^{®}), Fluocinonid (Topsym^{®}), Halometason (Sicorten^{®} plus), Methylprednisolonaceponat (Advantan^{®}), Mometasonfuroat (Elocom^{®}), Fluticasonpropionat (Cutivate^{®}), Prednicarbat (Prednicutan^{®}) oder Clobetasolpropionat (Dermovate^{®}).

Bevorzugte Zusatzstoffe sind ausgewählt aus Konservierungsmitteln, insbesondere Sorbinsäure und deren Alkali-, vor allem Kaliumsalze, in bevorzugten Mengen wie 0,01 bis 0,2 Gew. %.

Weitere bevorzugte Zusatzstoffe sind gewählt aus hautwirksamen Substanzen, gewählt aus Mono-und /oder Triglyceriden (mittelkettig: Capron- (C6), Capryl- (C8), Caprin- (C10) und die Laurinsäure (C12)). oder Polyalkoholen oder Mischungen hiervon.

Weiterhin kann es von Vorteil sein, wenn ein Zusatzwirkstoff, insbesondere ein Antihistaminikum, vor allem topisches Antihistaminikum und / oder ein Glukortikoid-Wirkstoff enthalten sind wie z.B. Hydrocortison.

Besonders bevorzugte Zusatzstoffe sind gewählt aus einem oder mehreren Konservierungsmitteln, einem oder mehreren mittelkettigen Triglyceriden, einem oder mehreren Antihistaminikum und / oder einem oder mehreren Glukokortikoiden.

### Bevorzugte Ausführungsformen:

Bevorzugte Ausführungsformen erfindungsgemäß eingesetzter Cremezubereitungen sind folgende:

### Ausführungsform 1

Halbfeste Creme-Zubereitung zur topischen Anwendung bei Ektoparasiten / Ektoparasiten-Befall, vor allem Milben und bei der Behandlung von Krätze, wobei die Zubereitung umfasst: 38 Gew.% bis 73 Gew.% Wasser, 13 Gew.% bis 34 Gew.% eine oder mehrere unpolare Lipid-Kohlenwasserstoffkomponenten (KW), 6 Gew.% bis 15 Gew.% eines oder mehrerer Hilfsstoffe mit Emulgiereigenschaften (EMU), 0,01 bis 5 Gew.% Zusatzstoffe, ausgewählt aus Konditioniermitteln, Hautpflegestoffen, Zusatzwirkstoffen, sowie Mischungen derartigen Zusatzstoffe.

### Ausführungsform 2

Halbfeste Creme- Zubereitung zur topischen Anwendung bei Ektoparasiten / Ektoparasiten-Befall, vor allem Milben und bei der Behandlung von Krätze, wobei die Kohlenwasserstoffkomponente (KW) ausgewählt ist aus festem Paraffin, dünnflüssigem Paraffin, dickflüssigem Paraffin, weißem Vaselin, gelbem Vaselin, mikrokristallinem Paraffin oder Mischungen hiervon.

### Ausführungsform 3

Halbfeste Cremezubereitung für die erfindungsgemäße Anwendung bei der Bekämpfung von Ektoparasiten, vor allem Milben, und dadurch bedingten Hauterkrankungen wie Krätze, umfassend, vorzugsweise bestehend aus, 38 Gew.% bis 73 Gew.% Wasser, 13 Gew.% bis 34 Gew.% einer oder mehrerer insbesondere unpolarer Kohlenwasserstoffe - Gemische (= unpolare Lipid)- Komponente(n) (KW) aus der Gruppe, umfassend Paraffin, Vaselin, insbesondere weißes Vaselin, gelbes Vaselin, dickflüssiges Paraffin, mikrokristallines Paraffin, Hartparaffin, dünnflüssiges Paraffin oder Mischungen hiervon, 6 Gew. % bis 15 Gew. % eines oder mehrerer Hilfsstoffe mit Emulgiereigenschaften (EMU), ausgewählt aus C₁₀-C₂₈-Fettalkoholen und C₁₀-C₂₈--Fettalkoholsulfaten und / oder deren -Sulfatsalzen, sowie Mischungen hiervon, 0 bis 7%, vorzugsweise 0 bis 5%, vor allem 0,01 bis 5 Gew.% Zusatzstoffe, ausgewählt aus Stabilisatoren, Hautpflegestoffen, Zusatzwirkstoffen oder Mischungen derartiger Zusatzstoffe, sowie als antiparasitären Wirkstoff 8 bis 13 Gew.% Permethrin (WS), bezogen jeweils auf das Gesamtgewicht der Zubereitung, aufweist, wobei das Verhältnis von Lipidkomponente (KW) zu (WS) Wirkstoff Permethrin 1,5 bis 2,7, insbesondere 2,0 bis 2,2, bezogen auf Gew.% von (KW) und (WS) in der Gesamtzubereitung, beträgt.

### Ausführungsform 4:

Zubereitung zur erfindungsgemäßen topischen dermalen Anwendung bei Ektoparasiten / Ektoparasiten-Befall, vor allem Milben/Krätze, umfassend, oder bestehend aus: 30 Gew. % bis 65 Gew.% Wasser; 8 Gew.% bis 13, insbesondere 8 Gew.%-12 Gew.%, vor allem 9 bis 12 Gew.%, ganz besonders 10 Gew.% Permethrin (WS), jeweils bevorzugt als 25: 75 cis/trans Gemisch,
18 Gew. % bis 30 Gew.%, Kohlenwasserstoffkomponente (KW), ausgewählt einem Vaselin, vor allem aus weißem Vaselin, gelbem Vaselin, dickflüssigem Paraffin, dünnflüssigem Paraffin oder Mischungen aus einem oder mehreren dieser Kohlenwasserstoffe (KW), 7-13 Gew.% Hilfsstoff mit Emulgatoreigenschaften (EMU), ausgewählt aus Emulgierendem Cetylstearylalkohol, Laurylalkohol, Cetylalkohol, Stearylalkohol, Cetylsterylalkohol, Natriumlaurylsulfat, Natriumcetylstearylsulfat oder Mischungen hiervon, insbesondere emulgierender Cetylstearylalkohol, Typ A oder Typ B, (EMU), 0 bis 2 Gew.%, vor allem 0,01 bis 2 Gew. %, bevorzugt 0,05 bis 1,5%, Zusatzstoffe, vor allem Konservierungsmittel, wobei das Verhältnis von (KW) zu (WS) 1,5 bis 2,7, oder auch 1,9 bis 2,3 oder 1,8 bis 2,4, insbesondere 2,0 bis 2,2 beträgt.

### Ausführungsform 5

In einer weiteren geeigneten Ausführungsform umfasst, insbesondere besteht die Creme-Zubereitung zur topischen dermalen Anwendung bei Ektoparasiten / Ektoparasiten-Befall, vor allem Milben/Krätze Anwendung aus:
50 bis 65 Gew.% Wasser, 9-12 Gew.%, insbesondere 10 Gew.% Permethrin-Wirkstoff (WS) jeweils bevorzugt mit 25: 75 % cis / trans Anteil; 15 bis 26 Gew. % flüssigen oder halbfesten Kohlenwasserstoffen oder Mischungen hiervon (KW) ausgewählt, aus weißem Vaselin, gelbem Vaselin, dickflüssigem Paraffin, mikrokristallinem Paraffin, Hartparaffin, dünnflüssigem Paraffin oder Mischungen hiervon, insbesondere Mischungen hiervon wie vor allem Vaselin weiß (Vaselinum album) im Gemisch mit Paraffin liquid, 8 bis 13 Gew.%, vor allem 7 bis 11 Gew. % Hilfsstoff mit Emulgiereigenschaften (EMU), ausgewählt aus emulgierendem Cetylstearylalkohol Typ A oder Typ B, und 0,01 bis 1 Gew. % Konservierungsstoff, insbesondere Sorbinsäure im Gemisch mit Natrium- oder Kaliumsorbat,
wobei das Gewichtsverhältnis (KW) zu (WS) 1,5 bis 2,7, oder auch 1,9 bis 2,3 oder 1,8 bis 2,4, insbesondere 2,0 bis 2,2, ganz besonders 2,1 beträgt.

In weiteren bevorzugt verwendeten Ausführungsformen sind folgende Komponenten vorhanden:

### Ausführungsform 6:

Die hier eingesetzte Zubereitung zur erfindungsgemäßen zur topischen dermalen Anwendung bei Ektoparasiten / Ektoparasiten-Befall, vor allem Milben/Krätze umfasst:
25 Gew. % (KW), ausgewählt aus Vaselin (weiß und / oder gelb), dickflüssiges Paraffin (liquid), oder Mischungen hiervon und 12 Gew.% Permethrin (WS).

### Ausführungsform 7:

Die Zubereitung zur erfindungsgemäßen zur topischen dermalen Anwendung bei Ektoparasiten / Ektoparasiten-Befall, vor allem Milben/Krätze umfasst:
21 Gew. % (KW), ausgewählt aus Vaselin (weiß und / oder gelb), dickflüssiges Paraffin (liquid), white soft Paraffin, mikrokristallines Paraffin, insbesondere Mischungen hiervon und 10 Gew.% Permethrin (WS).

### Ausführungsform 8:

Die Zubereitung zur erfindungsgemäßen zur topischen dermalen Anwendung bei Ektoparasiten / Ektoparasiten-Befall, vor allem Milben/Krätze Anwendung umfasst:
34 Gew. % (KW), ausgewählt aus Vaselin (weiß und/oder gelb), dickflüssiges Paraffin (liquid), white soft Paraffin, oder Mischungen hiervon und 13 Gew.% Permethrin (WS).

### Ausführungsform 9:

Die Zubereitung zur erfindungsgemäßen zur topischen dermalen Anwendung bei Ektoparasiten / Ektoparasiten-Befall, vor allem Milben/Krätze Anwendung umfasst:
13 Gew.% (KW), ausgewählt aus Vaselin (weiß und/oder gelb), dickflüssiges Paraffin (liquid), white soft Paraffin, oder Mischungen hiervon und 8 Gew.% Permethrin (WS) .

### Ausführungsform 10:

Die Zubereitung zur erfindungsgemäßen zur topischen dermalen Anwendung bei Ektoparasiten / Ektoparasiten-Befall, vor allem Milben/Krätze Anwendung umfasst:
17 Gew.% (KW), ausgewählt aus Vaselin (weiß und/oder gelb), dickflüssiges Paraffin (liquid), white soft Paraffin, oder Mischungen hiervon und 9 Gew.% Permethrin (WS) .

Soweit nicht anders angegeben, umfassen, oder bestehen aus, die Ausführungsformen 6 bis 10 weiterhin die / den erwähnten Mengen an Wasser (wie 38 bis 73 Gew. % oder bevorzugte Bereiche) und Hilfsstoff (EMU) (6 bis 15 Gew.% oder bevorzugte Bereiche) sowie Zusatzstoffe, soweit vorhanden (0 bis 7 Gew.% oder bevorzugte Bereiche).

In allen vorgenannten Creme-Zubereitungen, vor allem auch den bevorzugten Ausführungsformen, wie sie erfindungsgemäß verwendet werden können, beträgt der Anteil an (EMU) wie oben definiert, vor allem emulgierendem Cetylstearylalkohol Typ A oder Typ B, bevorzugt 8 bis 10 Gew.%, insbesondere 9 Gew.%.

In den erfindungsgemäß verwendeten Zubereitungen, vor allem in den oben beschriebenen bevorzugten Ausführungsformen 1 bis 8, ist es weiterhin von Vorteil, wenn das Gewichtsverhältnis von Emulgierendem Hilfsstoff (EMU), insbesondere emulgierender Cetylstearylalkohol Typ A oder Typ B, zu Wirkstoff (WS) 0,8 bis 1,3, oder auch 0,9 bis 1,1, insbesondere 0,9 beträgt.

In den eingesetzten Creme-Zubereitungen und / oder den bevorzugten Varianten hiervon sind weiterhin bevorzugt ca. 45 bis 65 Gew. % Wasser und / oder 16 bis 25 Gew.% LipidKomponenten (KW) vorhanden.

Die erfindungsgemäß eingesetzte Zubereitung und auch die bevorzugten Ausführungsformen wie verwendet umfassen vorzugsweise keine organischen Lösungsmittel, gewählt aus N-Met-hylpyrrodidon oder auch andere N-Alkylpyrrolidone wie N-C₈-C₁₈-Alkyl-Pyrrolidone oder N-C₁-C₄-Alkyl-Pyrrolidone und / oder auch keine aromatischen Lösungsmittel wie Biphenyle, Toluole wie oben im Stand der Technik beschrieben.

### Herstellung und Beispiele

Die erfindungsgemäße Zubereitung wird in an sich bekannter Weise hergestellt aus Wasserphase und Lipidphase. Für die Lipidphase werden die Kohlenwasserstoffe und der Emulgator (z.B. Emulgierender Cetylstearylalkohol, dickflüssiges Paraffin und Vaselin) zunächst geschmolzen. Der erwärmte Wirkstoff wird zugegeben und homogenisiert. Für die Wasserphase wird das erwärmte Wasser ggf. mit vorhandenen Zusätzen wie gelöstem Konservierungsmittel (z.B. Sorbinsäure) bereitgestellt. Unter Rühren wird dann die Wasserphase in die Fettphase eingearbeitet. Nachdem die fertige Creme abgekühlt ist, ist die Herstellung abgeschlossen.

Es handelt sich insgesamt um insbesondere pharmazeutisch verträgliche Inhaltsstoffe, wobei keine komplexen Gemische bereitet werden müssen, aufgrund der geringen Anzahl an Einzelkomponenten. Die auf diese Weise bereitete Creme wird in Behältnisse/ Vorrichtungen wie Tuben, Kruken oder Spender zu üblicherweise 20 bis 100, insbesondere 30, 50 oder 60 g abgefüllt, aus denen die anmeldungsgemäße kutane Anwendung erfolgen kann.

Folgende Zubereitungen wurden wie geschildert hergestellt:
Beispiel 1: 10% Permethrin

| Inhaltsstoff (gemäß aktueller Ph. Eur) | Menge [g] | |
|---|---|---|
| Permethrin BPC cis:trans 25:75 | 100,0 | |
| Cetostearyl alcohol (type A), emul. | 90,0 | |
| Paraffin, liquid (dickflüssiges Paraffin) | 105,0 | |
| Paraffin white soft | 105,0 | |
| Sorbinsäure | 1,2 | |
| Gereinigtes Wasser | 598,8 | |
| Summe | 1000,0 | |

Beispiel 2: 8% Permethrin

| Inhaltsstoff (gemäß aktueller Ph. Eur) | Menge [g] | |
|---|---|---|
| Permethrin BPC cis:trans 25:75 | 80,0 | |
| Cetostearyl alcohol (type A), emul. | 90,0 | |
| Paraffin, liquid (dickflüssiges Paraffin) | 80,0 | |
| Paraffin, white soft | 100,0 | |
| Sorbinsäure | 1,2 | |
| Gereinigtes Wasser | 648,8 | |
| Summe | 1000,0 | |

Beispiel 3: 12% Permethrin

| Inhaltsstoff (gemäß aktueller Ph. Eur) | Menge [g] | |
|---|---|---|
| Permethrin BPC cis:trans 25:75 | 120,0 | |
| Cetostearyl alcohol (type A), emul. | 120,0 | |
| Paraffin, liquid (dickflüssiges Paraffin) | 125,0 | |
| Paraffin, white soft | 115,0 | |
| Sorbinsäure | 1,2 | |
| Gereinigtes Wasser | 518,8 | |
| Summe | 1000,0 | |

Vergleichsbeispiel 4: 5% Permethrin

| Inhaltsstoff (gemäß aktueller Ph.Eur.) | Menge [g] | |
|---|---|---|
| Permethrin BPC cis:trans 25:75 | 50,0 | |
| Cetostearyl alcohol (type A), emul. | 90,0 | |
| Paraffin, liquid (dickflüssiges Paraffin) | 105,0 | |
| Paraffin, white soft | 105,0 | |
| Sorbinsäure | 1,2 | |
| Gereinigtes Wasser | 648,8 | |
| Summe | 1000,0 | |

### Anwendung

Die erfindungsgemäße Zubereitung wird aus geeigneten Vorrichtungen wie Tuben, Kruken oder Spender direkt auf die Haut des Betroffenen, in der Regel des gesamten Körpers (unter Aussparung lediglich der Mund- und Augenareale) aufgetragen. Dabei ist vor allem auf die vollständige Abdeckung der Prädeliktionsstellen, also der Hände und Füße, sowie der Haut um die Finger- und Zehennägel und des Intimbereiches und der Perianalregion zu achten. Dazu werden üblicherweise in Abhängigkeit von Körpergröße und Gewicht 25-60 g Creme verwendet einmalig aufgetragen werden. Die Zubereitung ist hautverträglich und zieht rasch ein und soll üblicherweise mindestens einige Stunden auf der Haut verbleiben, genauer, mindestens 8 Stunden. Bei ausbleibender Heilung bzw. nicht vollständiger Elimination z. B. der Skabiesmilben wird die Behandlung üblicherweise nach 7 bis 14 Tagen wiederholt.

Die Wirksamkeit wird anhand der nachfolgend beschriebenen Untersuchungen in den Anwendungsbeispielen A und B näher erläutert.

### Anwendungsbeispiel A: Hautpenetrationsuntersuchungen

Zur Untersuchung der Hautpenetration der erfindungsgemäßen Zubereitung wurde in einem spezialisierten Labor eine Studie an Humanhaut durchgeführt, bei der die Penetration in die Haut, insbesondere im Stratum Corneum im Vergleich zur herkömmlichen 5%igen Permethrincreme bestimmt wurde.

### Angewendete Formulierungen:

Permethrin 5 % Creme gemäß Vergleichsbeispiel 4
Permethrin 10 % Creme gemäß Beispiel 1

Von den Formulierungen wurden jeweils 20 mg aufgetragen, außer bei der ebenfalls durchgeführten Doppelapplikation von Permethrin aus der 5%igen Creme, gemäß Vergleichsbeispiel 4, 2x20 mg = 40 mg appliziert wurden. Die Analyse der jeweiligen Hautproben erfolgte durch Bestimmung der radioaktiven Zerfallsrate mittels Szintillatonszähler (TRICARB). Dafür wurde den Untersuchungsformulierungen vor Applikation radiaoaktiv-3markiertes (H)-Permethrin zugemischt (20 µCi je 20 mg Formulierung).

### Versuchsbedingungen:

Für jede Testformulierung wurden drei Humanhautproben, d. h. von drei verschiedenen Spendern untersucht. Jede Hautprobe ergibt drei Stanzbiopsien zu drei verschiedenen Zeitpunkten, d. h. insgesamt 27 Proben je Testformulierung. Für alle Formulierungen wurde die Haut der jeweils gleichen Spender verwendet.

### Präparation der Haut:

Die Untersuchungen wurden an exzidierter, humaner Brusthaut (3 verschiedene Spender) durchgeführt. Die Gewebeschnitte wurden postoperativ mit Tupfern und isotonischer NaCl-Lösung gereinigt. Das subkutane Fettgewebe wurde mechanisch seziert und verworfen. Zum Untersuchungszeitpunkt wurden die Hautstücke (Ø 20 mm; Fläche: 3,1416 cm²) bei Raumtemperatur vollständig aufgetaut und die Oberfläche mit Tupfern getrocknet. Die Bestimmung der Hautintegrität erfolgte mittels Corneometer.

### Versuchsdurchführung

Als Akzeptor wurde destilliertes Wasser verwendet. Die einzelnen Hautproben wurden auf Filtergaze aufgetragen und auf die auf 32 °C vorgewärmte Diffusionszelle gelegt (direkter Kontakt zwischen Filtergaze und Akzeptorflüssigkeit). Die Dicke der Diffusionsschicht wurde durch kontinuierliches Rühren verringert und somit physiologische Verhältnisse simuliert, indem permeierte Wirkstoffanteile stetig entfernt wurden (Sink-Bedingungen mit Glasabdeckung als Verdunstungsschutz). Nach homogenen Applikation der Prüfformulierung auf die

Hautproben folgt eine Inkubationszeit von 100 Minuten, nach der die restliche Formulierung mit einem Tupfer entfernt wurde. Danach wurden die Hautproben entnommen und drei Biopsien mit einer Größe von Ø 6 mm (0,848 cm²) aus der Haut ausgestanzt. Die Stanzbiopsien wurden anschließend bei -40ºC eingefroren und unter Verwendung eines Kühlmikrotoms horizontal geschnitten. Zur Bestimmung des Konzentrations-Zeit-Profils wurden die Hautschichten, die für die Penetrationsbestimmung relevant sind nach folgendem Schema präpariert:
Stratum corneum: SC: 20 Klebfilmabrisse von je 2 µm
Vitale Epidermis: EP: 10 Schnitte von je 20 µm
Dermis: DR: 15 Schnitte von je 40 µm

Für die anschließende Analyse wurden je 2 Klebfilmabrisse zu einer Fraktion vereint, nachfolgend zusammen bearbeitet und vermessen.

### Datenauswertung und Diskussion

Die Wirkstoffkonzentrationen in den verschiedenen Hautschnitten wurden unter Berücksichtigung von Schnittdicke, Fläche und applizierter Menge berechnet.

### Ergebnisse bezogen auf die erfindungsgemäße Zubereitung

Es konnte gezeigt werden, dass die höchste Permethrinkonzentration generell im Stratum corneum zu finden ist.

Da das natürliche Habitat der Skabiesmilben sich im Stratum corneum (SC) befindet, dort insbesondere in den mittleren und unteren Schichten und ggf. noch an der Grenze zur Epidermis (EP), sollen diese Hautschichten im Folgenden genauer betrachtet werden, wie in Tabelle 1 aufgezeigt.

**Tabelle 1**

| Hautschicht | Hauttiefe | | Permethrin 5% Creme | | Permethrin 10% Creme | | 2x Permethrin 5% Creme | |
|---|---|---|---|---|---|---|---|---|
| Probe | Mittlere Hauttiefe [µm] | | Cmean [mmol/L] | | Cmean [mmol/L] | | Cmean [mmol/L] | |
| SC4 | 8 | | 14,2321 | | 52,0851 | | 17,5427 | |
| SC5 | 10 | | 22,9727 | | 38,6382 | | 43,3344 | |
| SC6 | 12 | | 19,1771 | | 16,4989 | | 15,9880 | |
| SC7 | 14 | | 9,2907 | | 20,9011 | | 10,7788 | |
| SC8 | 16 | | 11,9998 | | 31,1494 | | 11,9491 | |
| SC9 | 18 | | 9,3283 | | 16,5139 | | 9,7920 | |
| SC10 | 20 | | 5,4676 | | 14,7149 | | 6,3927 | |
| EP1 | 40 | | 0,7470 | | 1,2468 | | 0,4691 | |
| EP2 | 60 | | 0,0827 | | 0,5762 | | 0,1719 | |
| SC 4-10 | 8-20 | | 92,4683 | | 190,5015 | | 115,7777 | |
| EP1-2 | 40-60 | | 0,8297 | | 1,8230 | | 0,6410 | |

**Tabelle 1:** Permethrin-Penetrationsprofile ins mittlere und untere Stratum Corneum (SC, jeweils mit Probennummer gemäß zunehmender Hauttiefe) sowie die obere Epidermis (EP, jeweils mit Probennummer gemäß zunehmender Hauttiefe) für Permethrin Creme 5%, Permethrin Creme 10% (erfindungsgemäß) und 2x Permethrin Creme 5% (doppelte Menge) nach 100 min Applikationszeit in Franzzell-Untersuchung mit Humanhaut (Cmean - mittlere Konzentration).

Wie hieraus ersichtlich, ist die Penetration von Permethrin 10% Creme in die mittleren und unteren Schichten des Stratum Corneums und der oberen Epidermis nicht nur deutlich höher als für Permethrin 5% Creme (Faktor > 2), was für lipophile Substanzen wie Permethrin nicht per se zu erwarten ist, sondern überraschenderweise auch deutlich höher als bei Auftragung der wirkstoffäquivalenten Menge (2x Permethrin 5% Creme). Hier zeigt sich für Permethrin 10% Creme ein überproportionaler Effekt von > +50% für die Stratum-Corneum-Schichten 4-10, bei Betrachtung nur der untersten SC-Schicht (SC10) sogar von >+100 %, trotz hier gleicher Wirkstoffmengenapplikation. Ein ähnliches Bild zeigt sich in der oberen Epidermis (EP1-2), mit ebenfalls > +100% für Permethrin Creme 10% gegenüber Permethrin 5% Creme als auch 2x Permethrin 5% Creme.
Anwendungsbeispiel B: Klinische Testung

### Methodik und Durchführung:

Zur Überprüfung der klinischen Wirksamkeit der patentgemäßen Permethrin 10% Creme bei durch Milbennachweis belegter und fachärztlich bestätigter Skabies wurde im Rahmen einer größeren prospektiven klinischen Testung bei Versagen der Primärtherapie mit Permethrin 5% Creme (nach Ganzkörper-Anwendung durch medizinisches Fachpersonal und Schulung zu den notwendigen Hygienemaßnahmen, Menge ad libidum) Patienten im Alter zwischen 8 und 85 Jahren in einem randomisierten Setting insbesondere auch eine Behandlung mit Permethrin 10% Creme im direkten Vergleich mit der 2fachen Anwendung von Permethrin 5% Creme zugewiesen. Die Applikation erfolgte im Ganzkörper-Setting (inkl. Kopf unter Aussparung des Gesichtes bzw. der Mund- und Augenpartien) mit jeweils 60 g Creme durch geschultes medizinischen Fachpersonal in dermatologischen Kliniken (für die 2fach-Anwendung 2x binnen 24 Stunden). Alle Patienten erhielten somit die gleiche Wirkstoffmenge. Als Therapieerfolg wurde die vollständige Eradikation aller Milben festgelegt, also ein negativer Skabiesmilbennachweis nach dermatologischer Ganzköperinspektion durch geschulte dermatologische Fachärzte. Die Überprüfung der Wirksamkeit erfolgte an Tag 14 nach Applikation. Sofern weiterhin Skabiesmilben nachweisbar waren, wurde bei den Patienten, die Permethrin 10% Creme erhalten hatten, eine weitere Behandlung mit Kontrolle nach 14 Tagen (Tag 28) angeschlossen.

### Ergebnisse und Diskussion:

Es ergaben sich folgende in Tabelle 2 dargestellte Ergebnisse:

### Tabelle 2

**Tabelle 2: Klinischer Therapieerfolg / Eradikation aller Skabiesmilben für 2x Permethrin 5% Creme und Permethrin 10% Creme (* gleiche Patienten), N= Anzahl der Patienten.**

| Anwendung / Konzentration | Therapieerfolg Tag 14 | | | Therapieerfolg Tag 28 | | |
|---|---|---|---|---|---|---|
| | N | Erfolg (n) | Erfolg (%) | N | Erfolg (n) | Erfolg(%) |
| 2x Permethrin 5% Creme (Vgl.) | 9 | 1 | 11,1 | | | |
| 1× Permethrin 10 % Creme (Erf.) | 12 | 4 | 33,3 | | | |
| 2x Permethrin 10% Creme (Erf.) | | | | 12* | 6 | 50 |

Die Testdaten zeigen, dass trotz Erhöhung der Auftragsmenge (obligat 60 g gegenüber der zuvor versagenden Standardanwendung mit Permethrin 5% Creme) und doppelter Anwendung binnen 24 h die Behandlung mit Permethrin 5% Creme kaum einen klinischen Effekt auf die Skabiesmilben zeigt (11% Therapieerfolg). Demgegenüber zeigt die einmalige Anwendung von Permethrin 10% Creme trotz Applikation der gleichen Wirkstoffmenge überraschenderweise einen (gegenüber Permethrin 5% Creme) überproportionalen, 3-fachen Effekt und damit die Überlegenheit der neuen, erfindungsgemäßen Formulierung. Dies ist umso bemerkenswerter als es sich um ein zuvor mit dem gleichen Wirkstoff (Permethrin) erfolglos behandelte Patienten handelt. In diesem Zusammenhang ist auch der zusätzliche Effekt der 2. Behandlung mit Permethrin 10% Creme positiv hervorzuheben. Die insgesamt mit maximal 2 Anwendungen erzielte Wirksamkeit von 50% ist ebenfalls überraschend und zeigt den Zugewinn an möglicher Wirksamkeit, der nach Versagen einer regulären Permethrintherapie durch die erfindungsgemäße 10%ige Permethrin Creme erreicht werden kann. Interessant ist dabei auch, dass von den geheilten Patienten immerhin 2 von 3 (67%) bereits mit einer Anwendung von Permethrin 10% Creme zum Therapieerfolg geführt werden können.

## Patentansprüche

1. Halbfeste Creme- Zubereitung, umfassend Wasser, 13 Gew.% bis 34 Gew.% eines oder mehrerer unpolarer flüssige, halbfester und /oder fester Kohlenwasserstoff-(Lipid)-komponenten oder Gemischen hiervon (KW), ausgewählt aus einem oder mehreren Vaselinen, einem oder mehreren Paraffinen oder Mischungen hiervon, ein oder mehrere polare Hilfsstoffe mit Emulgatoreigenschaften (EMU), ausgewählt aus C₁₀-C₂₈- Fettalkoholen, C₁₀ -C₂₈ -Fettalkoholsulfaten, C₁₀ -C₂₈ -Fettalkoholsulfatsalzen sowie Mischungen hiervon, 0 bis 7 Gew. % Zusatzstoffe, sowie 8 Gew. % bis 13 Gew. % des Wirkstoffs Permethrin (WS), wobei das Verhältnis von (KW) zu (WS) 1,5 bis 2,7, bezogen auf Gew. % von (KW) und (WS) in der Gesamtzubereitung, beträgt, zur topisch dermalen Anwendung bei Ektoparasiten-Befall.

2. Halbfeste Creme-Zubereitung zur Verwendung nach Anspruch 1, wobei die Zubereitung umfasst: 38 Gew.% bis 73 Gew.% Wasser, 13 Gew.% bis 34 Gew.% eine oder mehrere unpolare Lipid-Kohlenwasserstoffkomponenten (KW), 6 Gew.% bis 15 Gew.% eines oder mehrerer Hilfsstoffe mit Emulgiereigenschaften (EMU), 0,01 bis 5 Gew.% Zusatzstoffe, ausgewählt aus Konditioniermitteln, Hautpflegestoffen, Zusatzwirkstoffen, sowie Mischungen derartigen Zusatzstoffe.

3. Halbfeste Creme- Zubereitung zur Verwendung nach Anspruch 1 oder 2, wobei die Kohlenwasserstoffkomponente (KW) ausgewählt ist aus festem Paraffin, dünnflüssigem Paraffin, dickflüssigem Paraffin, weißem Vaselin, gelbem Vaselin, mikrokristallinem Paraffin oder Mischungen hiervon.

4. Halbfeste Creme-Zubereitung zur Verwendung nach Anspruch 1, 2 oder 3, wobei das Gewichtsverhältnis (KW) zu (WS) 2,0 bis 2,2 beträgt.

5. Halbfeste Creme-Zubereitung zur Verwendung nach Anspruch 1, 2, 3 oder 4, wobei die Zubereitung umfasst: 30 Gew.% bis 65 Gew.% Wasser; 9 bis 12 Gew.% Permethrin (WS), (25:75 cis/trans Gemisch), 18 Gew. % bis 30 Gew. % KohlenwasserstoffKomponente(n) (KW), ausgewählt aus, weißem Vaselin, gelbem Vaselin, dünnflüssigem Paraffin, dickfüssigem Paraffin, und Mischungen dieser Kohlenwasserstoffkomponenten, , 7 Gew. % bis 13 Gew.% Hilfsstoff mit Emulgatoreigenschaften (EMU) , ausgewählt aus Emulgierendem Cetylstearylalkohol, Laurylalkohol, Cetylalkohol, Stearylalkohol, Cetylsterylalkohol, Natriumlaurylsulfat, Natriumcetylstearylsulfat oder Mischungen hiervon, 0,01 bis 2 Gew.% Zusatzstoffe, wobei das Gewichtsverhältnis von (KW) zu (WS) 1,9 bis 2,3, insbesondere 2,0 bis 2,1, beträgt.

6. Halbfeste Zubereitung zur Verwendung nach 1, 2, 3, 4 oder 5, wobei in der Zubereitung 10 Gew.% Permethrin (WS) vorhanden sind.

7. Halbfeste Zubereitung zur Verwendung nach Anspruch 1, 2, 3, 4, 5 oder 6, wobei 7 Gew. % bis 11 Gew.% Hilfsstoff mit Emulgatoreigenschaften (EMU), ausgewählt aus Emulgierendem Cetylstearylalkohol Typ A oder Emulgierendem Cetylstearylalkohol Typ B, vorhanden ist.

8. Halbfeste Zubereitung zur Verwendung nach Anspruch 1, 2, 3, 4, 5, 6 oder 7, wobei das Gewichtsverhältnis von Hilfsstoffen mit Emulgatoreigenschaften (EMU), insbesondere Emulgierender Cetylstearyalkohol Typ A oder Typ B, zu Permethrin Wirkstoff (WS) 0,8 bis 1,3 beträgt.

9. Halbfeste Zubereitung zur Verwendung nach einem der Ansprüche 1, 2, 3, 4, 5, 6, 7 oder 8, wobei 0,01 bis 1,5 Gew. % Zusatzstoffe, ausgewählt aus Konservierungsmitteln, Hautpflegestoffen, Zusatzwirkstoffen, oder Mischungen hiervon vorhanden sind.

10. Zubereitung zur Verwendung nach Anspruch 9, wobei als Zusatzstoff Sorbinsäure und / oder Kaliumsorbat vorhanden sind.

11. Halbfeste Zubereitung nach einem der Ansprüche 1, 2, 3, 4, 5, 6, 7, 8, 9, oder 10 zur Anwendung bei der dermal-topischen Behandlung von Milbenbefall / Krätze (Skabies) beim Menschen.

12. Halbfeste Creme- Zubereitung zur dermal topischen Verwendung nach Anspruch 11, wobei die Zubereitung aus 50 bis 65 Gew.% Wasser, 15- 26 Gew. % Kohlenwasserstoffkomponente (KW), ausgewählt aus weißem Vaselin, gelbem Vaselin, dickflüssigem Paraffin, mikrokristallinem Paraffin, Hartparaffin, dünnflüssigem Paraffin oder Mischungen hiervon, 7 bis 11 Gew. % Hilfsstoff mit Emulgiereigenschaften (EMU), ausgewählt aus emulgierendem Cetylstearylalkohol Typ A oder Typ B, 10 Gew. % Permethrin (WS), und 0,01 bis 1 Gew. % Konservierungsstoff besteht, wobei das Gewichtsverhältnis von (KW) zu (WS) 1,9 zu 2,3 beträgt.

13. Verwendung von Permethrin (WS) und einer Kohlenwasserstoffkomponenten aus unpolaren flüssigen, halbfesten und / oder festen aliphatischen Kohlenwasserstoffen oder Mischungen hiervon (KW), ausgewählt aus, Hartparaffin, weißem Vaselin, gelbem Vaselin, dickflüssigem Paraffin, dünnflüssigem Paraffin, mikrokristallinem Paraffin (= Hartparaffin) oder Mischungen hiervon, im Gewichtsverhältnis (KW) zu (WS) von 1,5 bis 2,7 zur Herstellung einer halbfesten Creme-Zubereitung mit einer gegenüber einer Standardzubereitung (5% Gew.% Wirkstoffkonzentration) verbesserten antiparasitären Wirkung gegen Krätzmilben/Skabies, umfassend 8 bis 13 Gew.% Permethrin, 38 bis 73 Gew.% Wasser, 13 bis 34 Gew.% Kohlenwasserstoffkomponente (KW), 6 Gew.% bis 15 Gew.% eines oder mehrerer Hilfsstoffe mit Emulgatoreigenschaften (EMU), ausgewählt aus C₁₀ - C₂₈- Fettalkoholen und C₁₀ - C₂₈- Fettalkoholsulfaten, C₁₀ - C₂₈- Fettalkoholsulfatsalzen, emulgierendem Cetylstearylalkohol Typ A oder Typ B sowie Mischungen hiervon, 0 bis 7 Gew.%, vor allem 0,01 bis 5 Gew.% Zusatzstoffen.

## Geänderte Patentansprüche

### Geänderte Patentansprüche gemäss Regel 137(2) EPÜ.

1. Halbfeste Creme- Zubereitung, umfassend Wasser, 13 Gew.% bis 34 Gew.% eines oder mehrerer unpolarer flüssige, halbfester und /oder fester Kohlenwasserstoff-(Lipid)-komponenten oder Gemischen hiervon (KW), ausgewählt aus einem oder mehreren Vaselinen, einem oder mehreren Paraffinen oder Mischungen hiervon, ein oder mehrere polare Hilfsstoffe mit Emulgatoreigenschaften (EMU), ausgewählt aus C₁₀-C₂₈- Fettalkoholen, C₁₀ -C₂₈ -Fettalkoholsulfaten, C₁₀ -C₂₈ -Fettalkoholsulfatsalzen sowie Mischungen hiervon, 0 bis 7 Gew. % Zusatzstoffe, sowie 8 Gew. % bis 13 Gew. % des Wirkstoffs Permethrin (WS), wobei das Verhältnis von (KW) zu (WS) 1,5 bis 2,7, bezogen auf Gew. % von (KW) und (WS) in der Gesamtzubereitung, beträgt, zur topisch dermalen Anwendung bei Ektoparasiten-Befall.

2. Halbfeste Creme-Zubereitung zur Verwendung nach Anspruch 1, wobei die Zubereitung umfasst: 38 Gew.% bis 73 Gew.% Wasser, 13 Gew.% bis 34 Gew.% eine oder mehrere unpolare Lipid-Kohlenwasserstoffkomponenten (KW), 6 Gew.% bis 15 Gew.% eines oder mehrerer Hilfsstoffe mit Emulgiereigenschaften (EMU), 0,01 bis 5 Gew.% Zusatzstoffe, ausgewählt aus Konditioniermitteln, Hautpflegestoffen, Zusatzwirkstoffen, sowie Mischungen derartigen Zusatzstoffe.

3. Halbfeste Creme- Zubereitung zur Verwendung nach Anspruch 1 oder 2, wobei die Kohlenwasserstoffkomponente (KW) ausgewählt ist aus festem Paraffin, dünnflüssigem Paraffin, dickflüssigem Paraffin, weißem Vaselin, gelbem Vaselin, mikrokristallinem Paraffin oder Mischungen hiervon.

4. Halbfeste Creme-Zubereitung zur Verwendung nach Anspruch 1, 2 oder 3, wobei das Gewichtsverhältnis (KW) zu (WS) 2,0 bis 2,2 beträgt.

5. Halbfeste Creme-Zubereitung zur Verwendung nach Anspruch 1, 2, 3 oder 4, wobei die Zubereitung umfasst: 30 Gew.% bis 65 Gew.% Wasser; 9 bis 12 Gew.% Permethrin (WS), (25:75 cis/trans Gemisch), 18 Gew. % bis 30 Gew. % KohlenwasserstoffKomponente(n) (KW), ausgewählt aus, weißem Vaselin, gelbem Vaselin, dünnflüssigem Paraffin, dickfüssigem Paraffin, und Mischungen dieser Kohlenwasserstoffkomponenten, , 7 Gew. % bis 13 Gew.% Hilfsstoff mit Emulgatoreigenschaften (EMU) , ausgewählt aus Emulgierendem Cetylstearylalkohol, Laurylalkohol, Cetylalkohol, Stearylalkohol, Cetylsterylalkohol, Natriumlaurylsulfat, Natriumcetylstearylsulfat oder Mischungen hier von, 0,01 bis 2 Gew.% Zusatzstoffe, wobei das Gewichtsverhältnis von (KW) zu (WS) 1,9 bis 2,3, insbesondere 2,0 bis 2,1, beträgt.

6. Halbfeste Zubereitung zur Verwendung nach 1, 2, 3, 4 oder 5, wobei in der Zubereitung 10 Gew.% Permethrin (WS) vorhanden sind.

7. Halbfeste Zubereitung zur Verwendung nach Anspruch 1, 2, 3, 4, 5 oder 6, wobei 7 Gew. % bis 11 Gew.% Hilfsstoff mit Emulgatoreigenschaften (EMU), ausgewählt aus einem emulgierendem Gemisch enthaltend Cetylstearylalkohol ((Hexadecan-1-ol) und Stearylalkohol (Octadecan-1-ol) pflanzlichen oder tierischen Ursprungs).

8. Halbfeste Zubereitung zur Verwendung nach Anspruch 7 wobei der Hilfsstoff mit Emulgatoreigenschaften (EMU) ein emulgierender Cetylstearylalkohol ist, der aus mindestens 80,0 Prozent Cetylstearylalkohol und 7,0 Prozent der ionischen O/W-Emulgatoren Natriumcetylstearylsulfat (Typ A) oder Natriumlaurylsulfat (Typ B) besteht.

9. Halbfeste Zubereitung zur Verwendung nach Anspruch 1, 2, 3, 4, 5, 6 oder 7, wobei das Gewichtsverhältnis von Hilfsstoffen mit Emulgatoreigenschaften (EMU) zu Permethrin Wirkstoff (WS) 0,8 bis 1,3 beträgt.

10. Halbfeste Zubereitung zur Verwendung nach einem der Ansprüche 1, 2, 3, 4, 5, 6, 7 oder 8, wobei 0,01 bis 1,5 Gew. % Zusatzstoffe, ausgewählt aus Konservierungsmitteln, Hautpflegestoffen, Zusatzwirkstoffen, oder Mischungen hiervon vorhanden sind.

11. Zubereitung zur Verwendung nach Anspruch 9, wobei als Zusatzstoff Sorbinsäure und / oder Kaliumsorbat vorhanden sind.

12. Halbfeste Zubereitung nach einem der Ansprüche 1, 2, 3, 4, 5, 6, 7, 8, 9, oder 10 zur Anwendung bei der dermal-topischen Behandlung von Milbenbefall / Krätze (Skabies) beim Menschen.

13. Halbfeste Creme- Zubereitung zur dermal topischen Verwendung, wobei die Zubereitung aus 50 bis 65 Gew.% Wasser, 15- 26 Gew. % Kohlenwasserstoffkomponente (KW), ausgewählt aus weißem Vaselin, gelbem Vaselin, dickflüssigem Paraffin, mikrokristallinem Paraffin, Hartparaffin, dünnflüssigem Paraffin oder Mischungen hiervon, 7 bis 11 Gew. % Hilfsstoff mit Emulgiereigenschaften (EMU), ausgewählt aus emulgierendem Cetylstearylalkohol Typ A oder Typ B, 10 Gew. % Permethrin (WS), und 0,01 bis 1 Gew. % Konservierungsstoff besteht, wobei das Gewichtsverhältnis von (KW) zu (WS) 1,9 zu 2,3 beträgt.

14. Halbfeste Creme-Zubereitung zur dermal topischen Verwendung nach Anspruch 3, umfassend Permethrin (WS) und einer Kohlenwasserstoffkomponente aus unpolaren flüssigen, halbfesten und / oder festen aliphatischen Kohlenwasserstoffen, oder Mischungen hiervon (KW), ausgewählt aus, Hartparaffin, weißem Vaselin, gelbem Vaselin, dickflüssigem Paraffin, dünnflüssigem Paraffin, mikrokristallinem Paraffin (= Hartparaffin), oder Mischungen hiervon, im Gewichtsverhältnis (KW) zu (WS) von 1,5 bis 2,7 weiterhin umfassend 8 bis 13 Gew.% Permethrin, 38 bis 73 Gew.% Wasser, 13 bis 34 Gew.% Kohlenwasserstoffkomponente (KW), 6 bis 15 Gew.% eines oder mehrerer Hilfsstoffe mit Emulgatoreigenschaften (EMU), ausgewählt aus C₁₀ - C₂₈- Fettalkoholen und C₁₀ - C₂₈- Fettalkoholsulfaten, C₁₀ - C₂₈- Fettalkoholsulfatsalzen, emulgierendem Cetylstearylalkohol Typ A oder Typ B sowie Mischungen hiervon, 0 bis 7 Gew.%, vor allem 0,01 bis 5 Gew.% Zusatzstoffen, wobei gegenüber einer Standardzubereitung (mit 5% Gew.% Wirkstoffkonzentration) eine verbesserte antiparasitäre Wirkung gegen Krätzmilben/Skabies vorliegt.
